# EUROPEAN PATENT APPLICATION

(11) **EP 2 586 773 A1**
(43) Date of publication of application: **01.05.2013**
(21) Application number: 11186817.0
(22) Date of filing: 27.10.2011
(51) Int. Cl.: C07D 239/56, C07D 405/12, C07D 487/04, A61K 31/519

(54) **Synthesis of Triazolopyrimidine Compounds**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to the field of organic synthesis and describes the synthesis of specific triazolopyrimidine compounds and intermediates thereof as well as related derivatives.

## Description

The present invention relates to the field of organic synthesis, in particular to the synthesis of specific triazolopyrimidine compounds and intermediates thereof as well as related derivatives.

### Background of invention

An important triazolopyrimidine compound is ticagrelor (**TCG**; Brilinta^{®}; 3-[7-[[(1*R*,2*S*)-2-(3,4-difluorophenyl)cyclopropyl]amino]-5-(propylthio)-3*H*-1,2,3-triazolo[4,5-d]pyrimidin-3-yl]-5-(2-hydroxyethoxy)-(1*S*,2*S*,3*R*,5*S*)-1,2-cyclopentanediol) having the following structural formula.

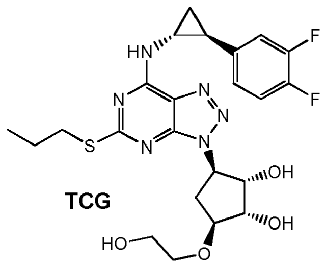

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

The synthesis of ticagrelor (**TCG**) is demanding. There are five to six known synthetic variants, which are described in the basic patent application WO 00/34283, an improved one in patent application WO 01/92263, and a further improved one in patent application WO 10/030224 respectively derived from the originator AstraZeneca, while two are published in a "deutero" patent application WO 11/017108 of Auspex Pharmaceuticals. Further, there is one synthetic path published in a scientific journal (Bioorg. Med. Chem. Lett. 2007, 17, 6013-6018).

The first synthesis of **TCG** as described in WO 00/34283 is depicted in scheme 1 below.

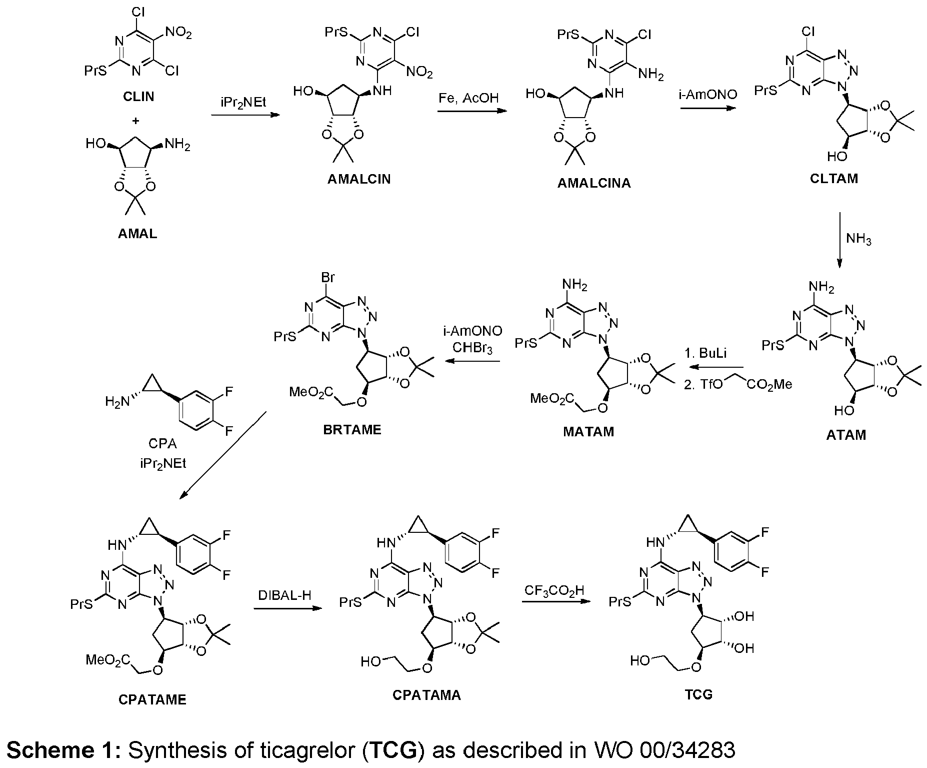

This nine step synthesis of ticagrelor (**TCG**) as described in WO 00/34283 (Scheme 1) starts with a reaction between **CLIN** and **AMAL.** In the presence of diizopropylethylamine (*i*Pr₂Net) **AMALCIN** is formed, which is in then reduced with iron (Fe) in acetic acid to **AMALCINA.** In the next step **CLTAM** is formed using isopentyl nitrite (*i*AmONO). Next, **ATAM** was prepared using ammonia, and side chain was introduced (**MATAM**) using *n*-butyllithium and methyl 2-(((trifluoromethyl)sulfonyl)oxy)acetate, which was previously prepared by reaction between methyl glycolate and triflic anhydride. In next step **BRTAME** is formed using iAmONO and CHBr₃, followed by the aromatic nucleophilic substitution of Br with **CPA** in the presence of *i*Pr₂NEt to form **CPATAME.** This is then reduced to **CPATAMA** using DIBAL-H. Deprotection of diol group in the presence of trifluoroacetic acid in the final step leads to **TCG.**

This synthetic path is very long (9 steps, not including reagents preparation) and uses toxic compounds like CHBr₃, triflic anhydride, and methyl 2-(((trifluoromethyl)sulfonyl)oxy)acetate.

An improved synthesis of ticagrelor (**TCG**) is described in WO 01/92263 (see Scheme 2). In this process the hydroxyethyl side chain is introduced at the beginning of the synthesis by a three step reaction path from **AMAL** to **AMALA,** which is then reacted with **CLINA** (prepared from **CLIDA**) in presence of triethylamine (Et₃N) to form **AMALCINAA.** The triazole ring of **CLTAM** is formed with NaNO₂ in acetic acid, and then Cl is exchanged with **CPA** to form **CPATAMA.** In the final step **TCG** is prepared via deprotection using **HCl.**

This improved process still has substantial length (7-8 steps). In **AMALA** synthesis the benzyloxycarbonyl protection (Cbz) is used, which is then removed in the third step using hydrogenation with Pd/C as a catalyst. Also, hydrogenation with Pt/C as a catalyst is used in the reduction of **CLIDA** to **CLINA.**

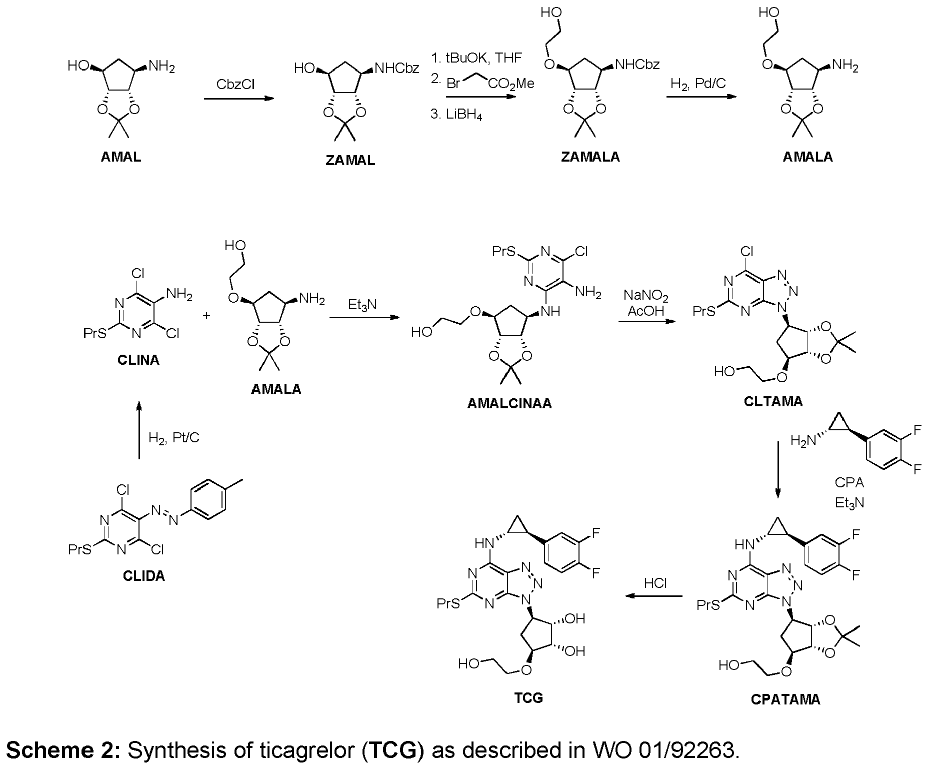

Another improved synthetic path is described in WO 10/030224 (Scheme 3). The key steps in this process are reduction of **CLIN** to **CLINA** or **AMALCINO** to **AMALCINAA** using hydrogen gas and platinum vanadium catalyst. The introduction of the hydroxyethyl side chain to **AMAL** to form **AMALA,** cyclization, substitution of Cl atom of **CLTAMA** with **CPA** and final acidic deprotection are the same as in WO 01/92263.

This further improved process to **TCG** has 8 reaction steps. Like in WO 01/92263, there are used the Cbz protecting group and heavy metals as catalysts like Pd, Pt and/or V.

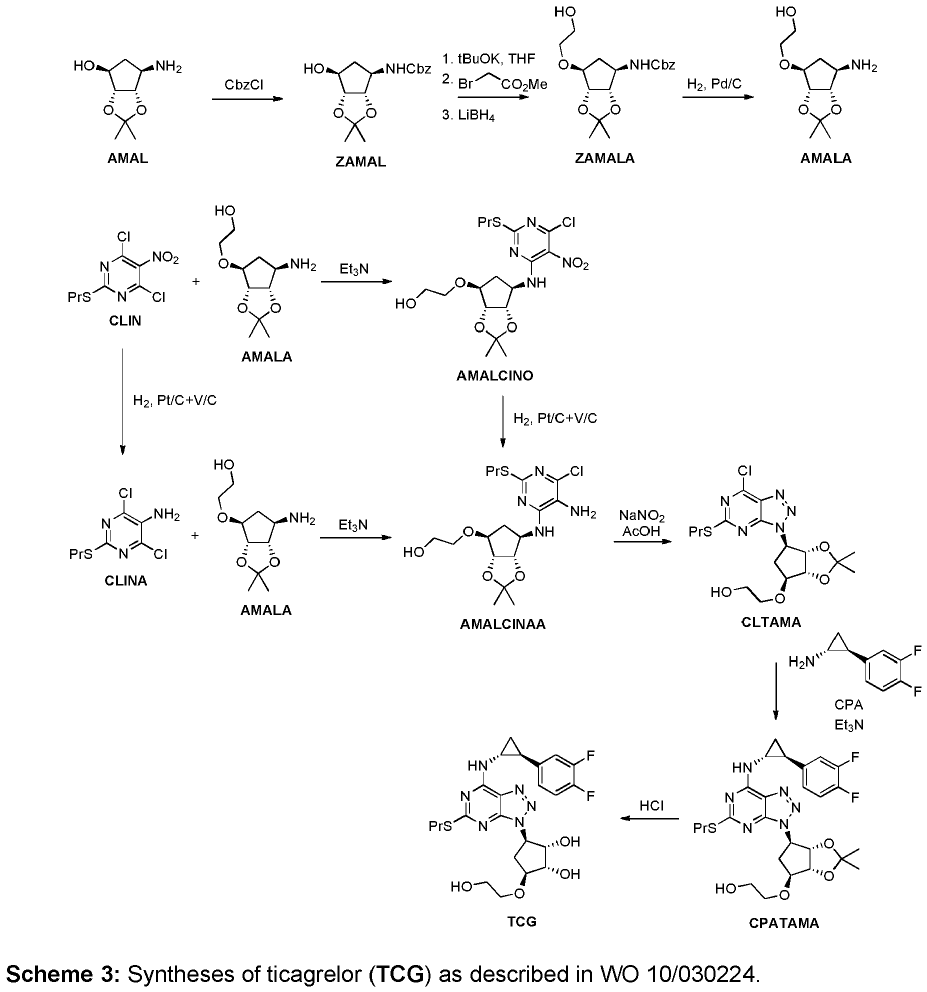

AstraZeneca published a synthetic path (Scheme 4) to ticagrelor (**TCG**) in Bioorg. Med. Chem. Lett. 2007, 17, 6013-6018. Intermediates in this process are similar to those described in WO 01/92263. There is difference in formation of triazolo ring of **CLTAMA** where iAmONO is used, and difference in deprotection in the last step.

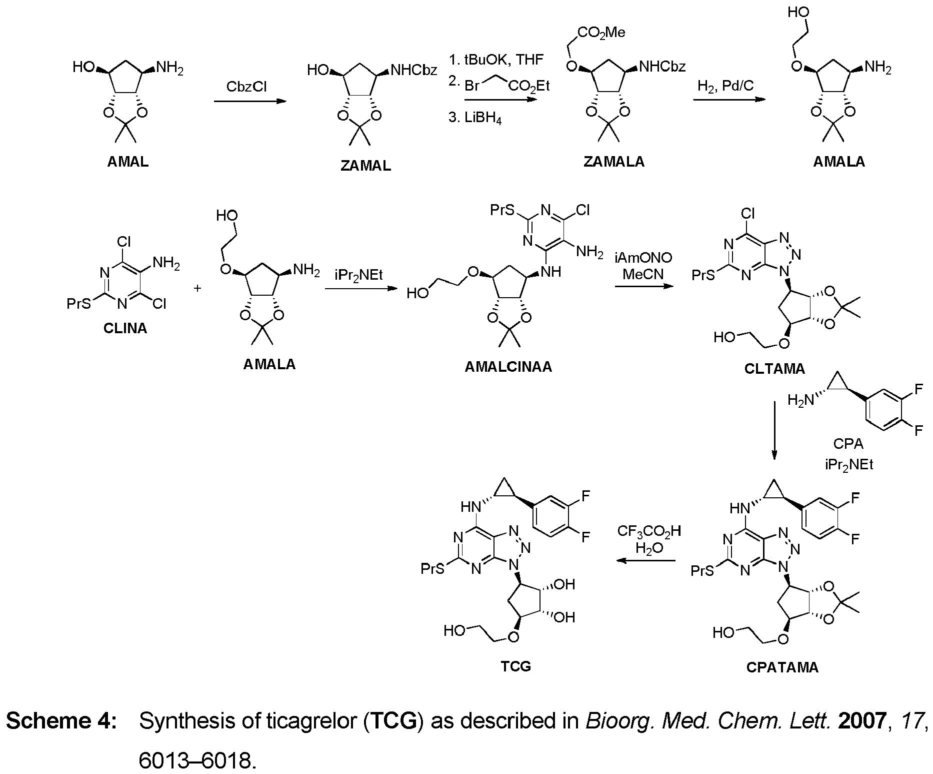

Another synthetic variant (Scheme 5) to ticagrelor (**TCG**) is described in WO 11/017108 by Auspex Pharmaceuticals. In nine step synthesis they prepared **AMALE** through deprotection of **ZAMALE** using hydrogen gas and Pd/C, which was then reduced to **AMALA** with LiAlH₄. **AMALCINO** was prepared without presence of base, further steps are similar to those published in WO 01/92263.

Still another synthetic variant (Scheme 6) to obtain ticagrelor with deuterated hydroxyethyl group (TCGD) is also described in WO 11/017108 by Auspex Pharmaceuticals. 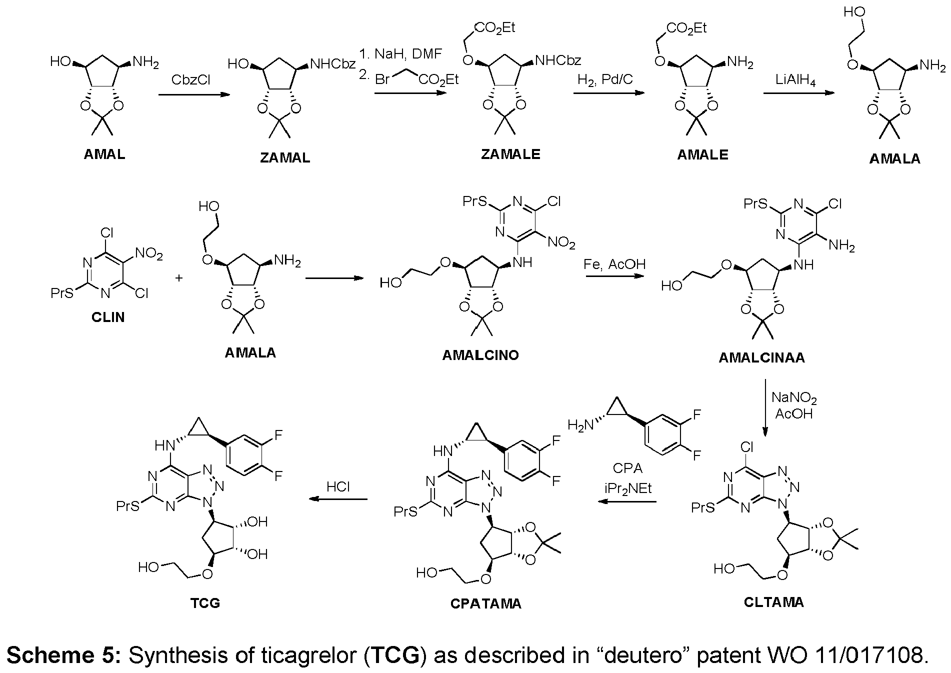 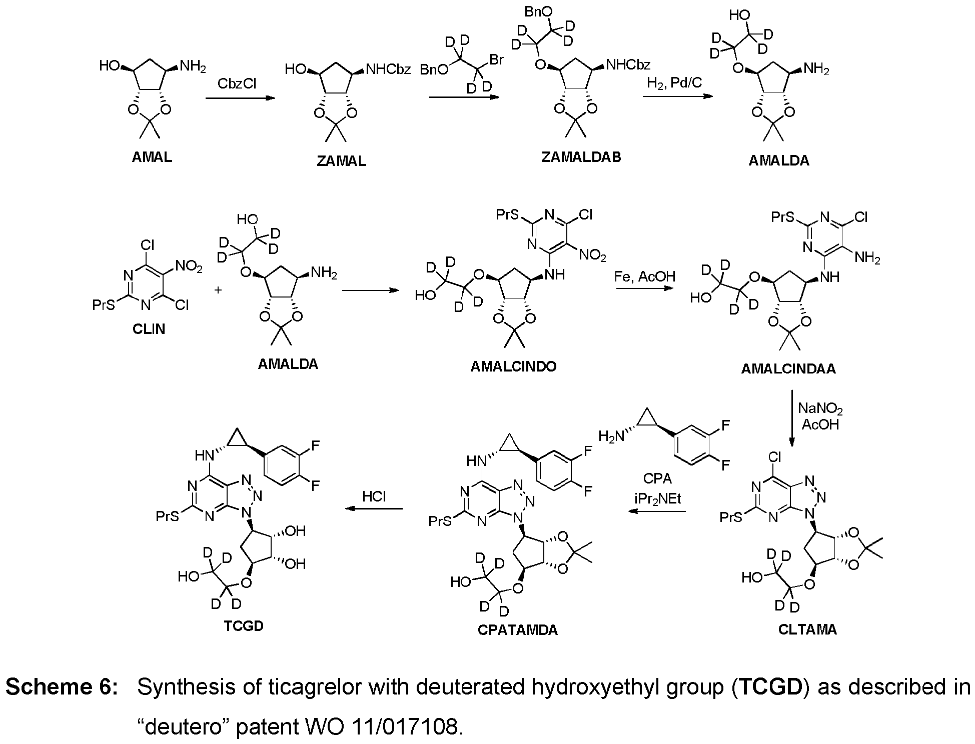 O-alkylation of the secondary alcohol functional group is often a demanding step for which a strong base such as sodium hydride is needed. The chemoselectivity problem arises in the presence of the reactive heteroaryl chloride functionality, because the oxy anion formed may attack the position of chloro atom ("selfarylation") leading to considerable amounts of byproducts (Scheme 7). In the known procedure, published in WO 00/34283 and represented in Scheme 1 the unwanted side reaction is avoided by first changing the reactive halogen to amino group, then conducting alkylation step and finally converting amino group back to halo group. Alternatively, hydoxyethyl group can be introduced by alkylation of cyclopentane part before heteroarylation as presented in upper parts of Schemes 2 to 6. However, in order to alkylate hydroxy group in the presence of an amino group the nitrogen atom must be protected. 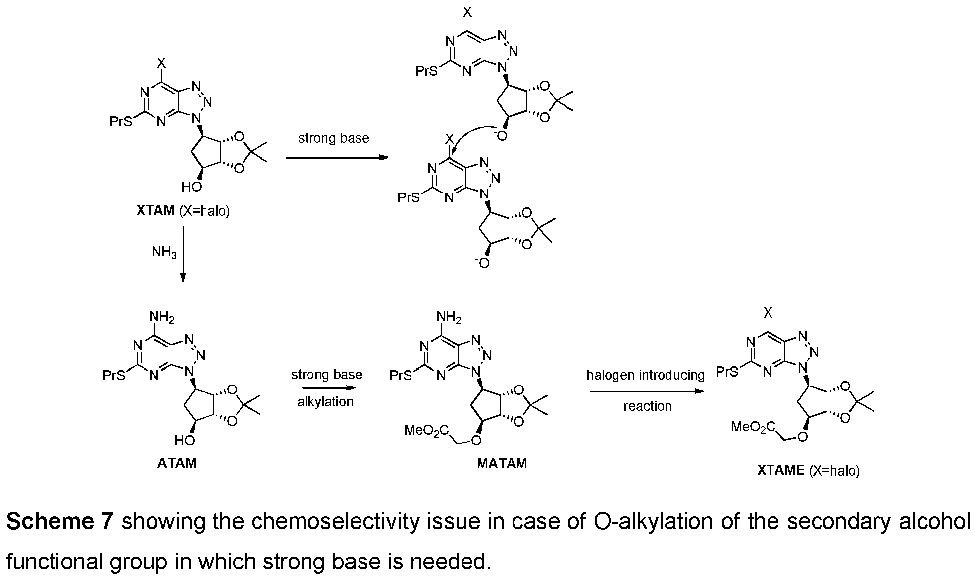 As becomes apparent from the above, a major drawback of the hitherto known synthesis schemes for the preparation of ticagrelor is that the synthesis is long.

### Summary of the Invention

The object of the present invention was to provide an industrially applicable and economically improved process for obtaining ticagrelor.

The present invention provides a process for the preparation of a compound of formula **VIII** 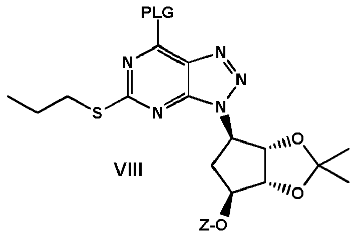 wherein PLG is a protecting-leaving group, and Z is hydroxyethyl or a group convertible to hydroxyethyl, the process comprising the steps of:
(i) providing a compound of formula **VII** 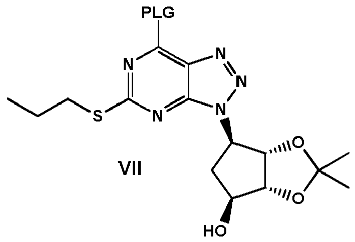 wherein PLG is defined as above, and
(ii) O-alkylating the compound of formula VII to obtain the compound of formula VIII.

The process defined above allows for preparation or synthesis of ticagrelor with an industrially applicable and economically improved process. Preferred embodiments will be described below. The present invention further provides novel compounds that are highly useful as key intermediates in the preparation or synthesis of ticagrelor.

### Description of the Invention and of Preferred Embodiments

Aspects, advantageous features and preferred embodiments of the present invention will be described in further detail below, noting however that such aspects, advantages features as well as embodiments and examples are presented for illustrative purposes only and shall not limit the invention in any way.

The introduction of the so called "protecting-leaving group" on the position 6 or 7 of the pyrimidine or triazolopyrimidine ring, respectively, of the intermediates in the synthesis of ticagrelor is a significant point of the present invention, which is a novel feature common to the key steps of the synthetic preparation of ticagrelor as well as to the intermediate compounds thereof. This crucial point, which distinguishes significantly over all prior art synthesis, allows that the hydroxyethyl group can be introduced in a later stage of the ticagrelor molecular assembly.

The "protecting-leaving group" (PLG) is a multipurpose single functional group which is able to serve as a protecting group in some chemical reactions, and then as a leaving group in a later reaction step. The role such a group takes depends on the reaction applied. There is a number of substitutions that can properly moderate the reactivity of the electron poor heteroaryl moiety while still allowing a subsequent nucleophilic aromatic substitution to occur. The PLG finely balances the reactivity in order to allow several transformations. Most suitably, the protecting-leaving group PLG is both, capable of acting as a protecting group in the O-alkylation reaction (ii) mentioned above, and capable of acting as a leaving group when it will be subjected to a nucleophilic substitution reaction. Halogen (notably Cl or Br) is excluded as PLG.

In particular, the process according to the present invention reduces the number of the required steps. Contrary to the prior art processes, in which "protection/deprotection scenario" is followed, the so called "protection/leaving scenario" does not need any deprotection step because the PLG is simply exchanged with a wished substituent, meaning that the number of required reaction steps is reduced by one step. At the same time, an increase in reaction selectivity is achieved.

A further significant advantage of the present invention resides in the possibility that several steps can be performed through one-pot conversions, without the need of isolation or separation of intermediate compounds, which one-pot system therefore constitutes a preferred embodiment of the present invention.

Accordingly, the possibility of reducing the number of required reaction steps, of increasing reaction selectivity, and of simplifying reactions respectively strongly contributes to provide an improved industrially applicable and economically beneficial process for obtaining triazolopyrimidine compounds and specifically ticagrelor.

According to a preferred embodiment, the compound of formula **VIIIa** is prepared from the compound of formula **VII,** for example by the O-alkylation with an alkyl haloacetate or alkyl sulfonyloxyacetate and the reduction of the so formed ester in order to build a 2-hydroxyethyl side chain. In this reaction the protecting ability of PLG is demonstrated. The leaving group propensity is finally employed in the substitution by amine, in which **VIIIa** is reacting with **IX** to give **CPATAMA.** See Scheme 8.

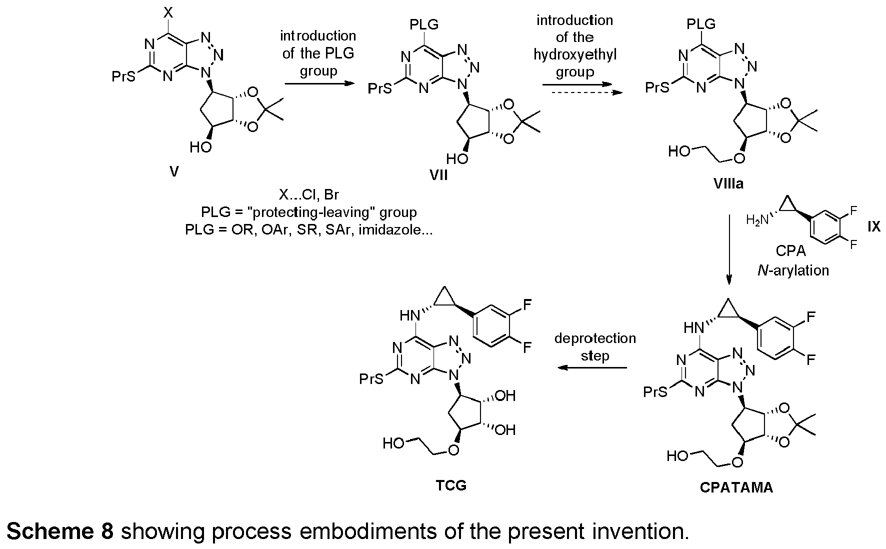

The PLG employed according to the present invention should provide properties that make the intermediates, for example a compound of formula **VII,** relatively stable in their deprotonated form at the reaction conditions required for the O-alkylation reaction, but at the same time it must impose reactivity for the subsequent reaction, in which an intermediate, for example a compound of formula VIIIa, reacts with an amine, for example a compound of formula IX (CPA). Optionally, the PLG can also be suitably selected to be stable under the ester group reduction conditions, when such a reaction is required in the building of the hydroxyethyl side chain.

It has been found that various functional groups possessing the above-mentioned properties can be efficiently introduced at different stages of ticagrelor synthesis. The protecting-leaving groups according to the present invention are selected from, but not limited to linear or branched C₁-C₆-alkoxy groups, optionally substituted with one or more aryl, heteroaryl, halo, C₁-C₄-alkoxy, C₁-C₄-alkylthio; aryloxy or substituted aryloxy; C₈-C₂₀-alkylthio, unsubstituted or substituted arylthio or heteroarylthio; N-azolyl groups, selected from unsubstituted or substituted 1-imidazolyl, 1-pyrrolyl, 1-pyrazolyl, 1-indolyl, 1-(1,2,3-triazolyl), 1-(1,2,4-triazolyl), 4-(1,2,4-triazolyl), 1-tetrazolyl, 2-tetrazolyl, 1-benzopyrazolyl, 1-benzimidazolyl, 1-benzotriazolyl, 5-carbazolyl, 4-aza, 5-aza, 6-aza, 7-aza, 4,5-diaza, 4,6-diaza, 4,7-diaza, 5,6-diaza, 5,7-diaza, or 6,7-diaza derivatives of 1-benzopyrazolyl, 1-benzimidazolyl, or 1-benzotriazolyl; N-amidyl groups selected from unsubstituted or substituted N-aryl-N-(C₁-C₆-alkanoyl)amino, 3-(2-oxo-1,3-oxazolidinyl), 3-(2-oxo-1,3-benzoxazolidinyl), 2-oxo-1-(1,2-dihydropyridyl), 2-oxo-1-(1,2-dihydroquinolyl), 2-oxo-1-(1,2-dihydroquinazolyl); 1-benzotriazolyloxy; azido, or cyano. The preferable representatives are presented below. The most preferable protecting-leaving groups are methoxy, benzyloxy, phenoxy or 1-imidazolyl groups.

| | | | | | | | | | |
|---|---|---|---|---|---|---|---|---|---|
| Preferred representative types and examples of the protecting-leaving group (PLG) | Alkoxy | | Aryloxy | | *N*-Azolyl | | *N*-Amidyl | | Thiolyl |
| 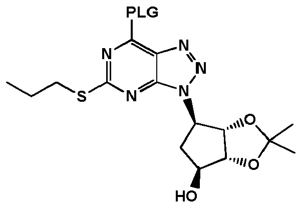 | 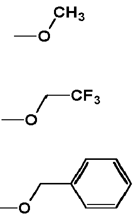 | | 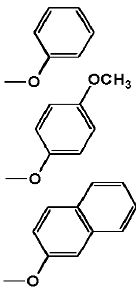 | | 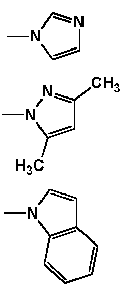 | | 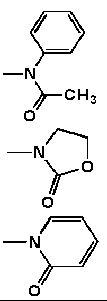 | | 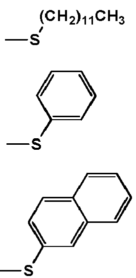 |

According to a preferred embodiment, the compound of formula VII is prepared by (i) providing a compound of formula **V** 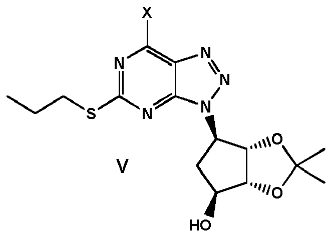 wherein X is Cl or Br, and
(ii) substituting **X** with PLG by reaction of a reagent PLG-H in the presence of a base, to obtain a compound of formula **VII.**

The compound of formula **V** can be prepared by
(i) providing a compound of formula **I'** 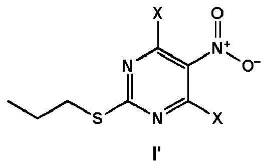 wherein X is Cl or Br,
(ii) reacting the compound of formula I' with a compound of formula **III** 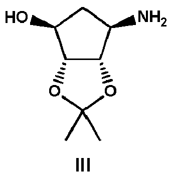 to obtain a compound of formula IV' 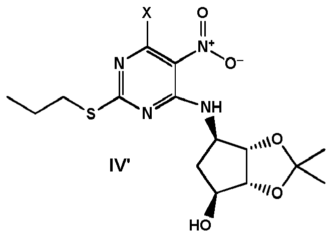 wherein X is as defined above,
(iii) reducing the nitro group of the compound of formula IV' to obtain a compound of formula IV" 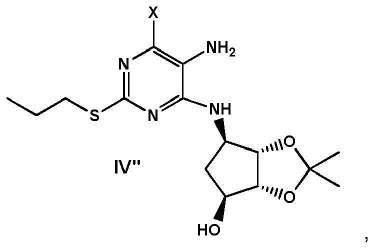 and
(iv) converting the compound of formula **IV"** into the compound of formula **V** by nitrosation.

In a characteristic part of the preferred embodiment of this invention shown in Scheme 9, PLG is introduced to heteroaryl halide **V,** for example heteroaryl chloride **CLTAM** (Va), by reaction of a reagent PLG-H in the presence of a base selected from the group consisting of metal or quaternary ammonium carbonates or phosphates, hydrogencarbonates, hydrogenphosphate, dihydrogenphosphate, hydroxides, alkoxides, hydrides, amides, alkyl metals or tertiary amines. The reaction is carried out at temperature from -20°C to the reflux temperature, preferably at room temperature. The reaction medium depends on the nature of PLG group and consists of solvents in which reactants are at least partially soluble, which are compatible with the applied bases and which are inert in cases in which they do not contribute in the substitution of halo atom. Such solvents are selected from the group of alcohols, cyclic ethers, ketones, nitriles, amides, halogenated hydrocarbons, cyclic or acyclic carbonates and esters or mixture of thereof, or partially with the solvents of other groups such sulphoxides, acyclic ethers, aromatic or aliphatic hydrocarbons or water. The preferred medium for introduction of C₁-C₆-alkoxy group is the corresponding C₁-C₆-alcohol and alkali metal carbonate, phosphate or alkoxide as a base. For example, the intermediate **MOTAM (Vila)** is prepared in methanol in the presence of potassium carbonate or sodium methoxide at room temperature. For the introduction of other groups, of which reagents cannot be used as a solvent, the preferred solvents are selected from cyclic ethers, such as tetrahydrofuran (THF), or methyltetrahydrofuran (MeTHF) or ketones, such as acetone. For example in the preparation of the aryl ether **FOTAM (VIIb)** the introduction of phenol is carried out in acetone in the presence of sodium carbonate, while in the characteristic example of preparation of the N-heteroarylimidazole IMTAM (VIIc) imidazole is introduced in THF or MeTHF and in the presence of triethylamine as a base. 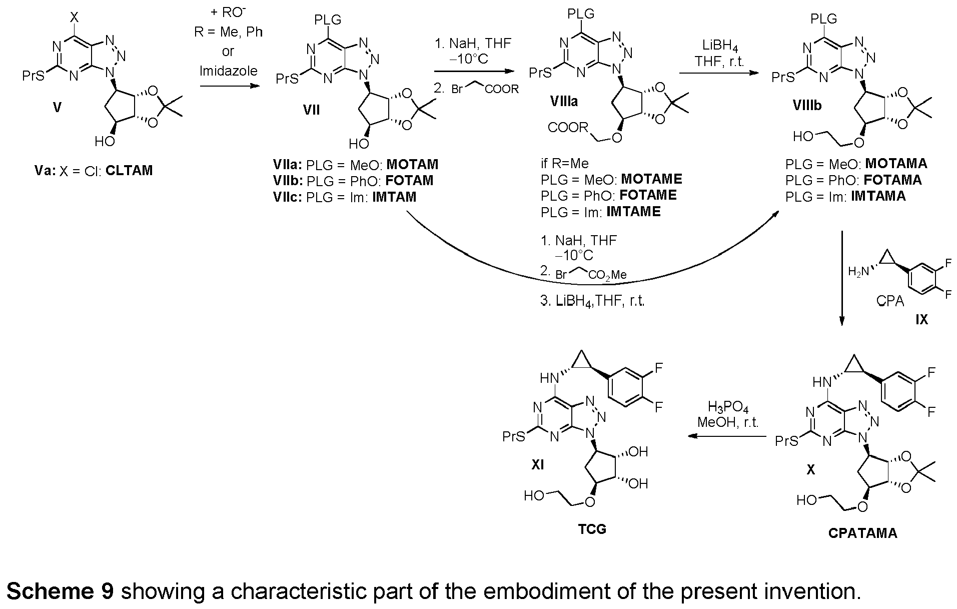 Alternatively, the compound of formula **VII** can be obtained by (i) providing a compound of formula **VI"** 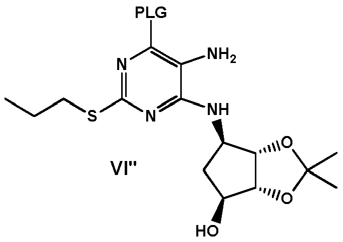 wherein PLG is defined as above, and (ii) converting the compound of formula VI" into the compound of formula VII by nitrosation.

The introduction of PLG can be applied in any of the steps of building-up the cyclopentyl substituted benzotriazole system as shown in a summary in Scheme 10. The nature of PLG determines in which particular step the introduction is the most convenient with respect to obtaining better yields. Introduction of PLG can be carried out in analogous reaction conditions as described above for the conversion of intermediate **V** to **VII,** optionally taking into consideration specialities of surrounding groups.

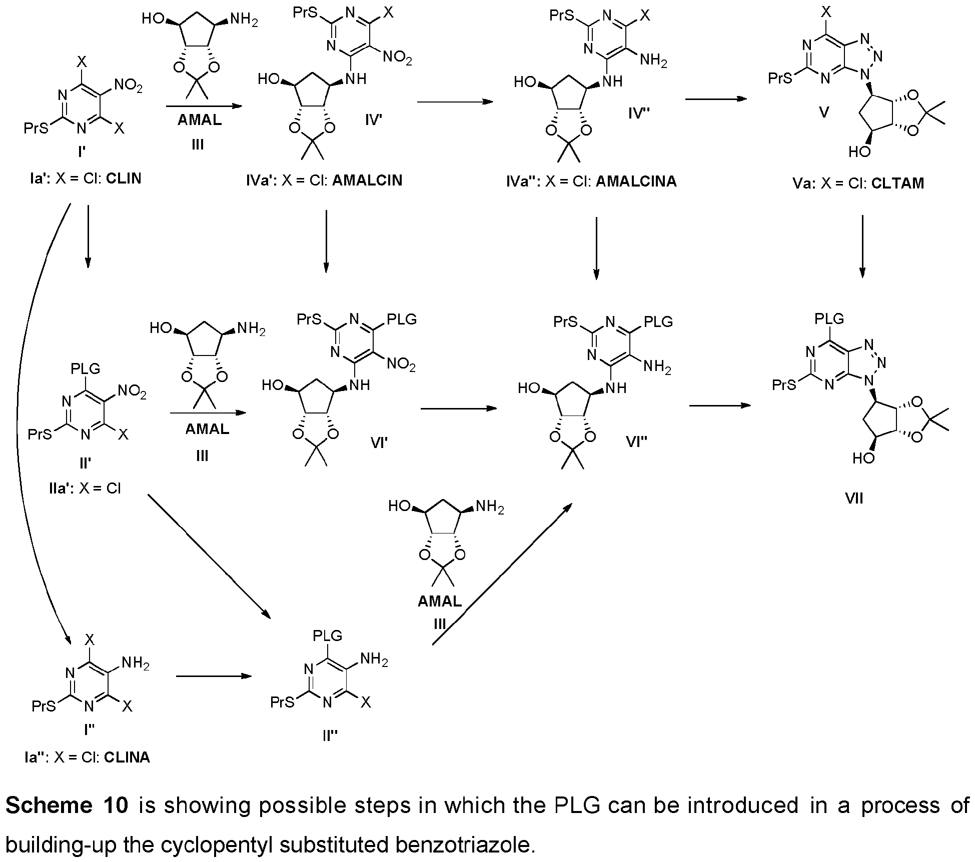

In another embodiment of the present invention the compound of formula **VI"** can be prepared by
(0-1) providing a compound of formula **IV"** 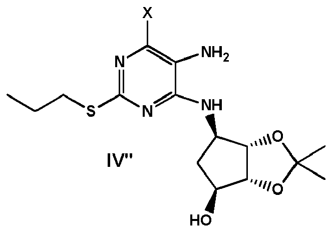 wherein X is Cl or Br, and
(0-2) substituting X with PLG by reaction of a reagent PLG-H in the presence of a base, to obtain a compound of formula **VI**"

**In** such cases, the compound of formula **IV"** is obtained as described above.

Alternatively, the compound of formula **VI"** can be obtained by carrying out the reduction of the nitro group to amino group of the intermediate already comprising the PLG, by comprising the steps of:
(0-1') providing a compound of formula **VI'** 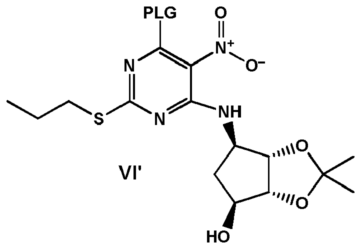
(0-2') reducing the nitro group of the compound of formula VI' to obtain a compound of formula VI"

The compound of formula VI' can be obtained from the compound of formula I' 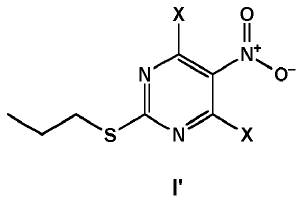 wherein X is Cl or Br,
either by first reacting the compound of formula I' with the compound of formula **III** 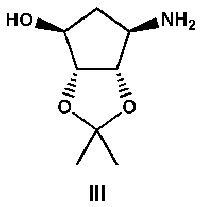 and subsequently substituting X with PLG by reaction of a reagent PLG-H in the presence of a base, or
by first substituting X with PLG by reaction of a reagent PLG-H in the presence of a base, and subsequently reacting the obtained formula **II'** with the compound of formula **III.**

In yet another embodiment the compound of formula **VI"** can be prepared by
(0-1 ") providing a compound of formula **II"** 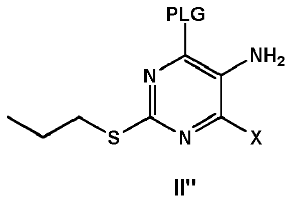 wherein X is Cl or Br,
(0-2") reacting the compound of formula II" with a compound of formula **III** 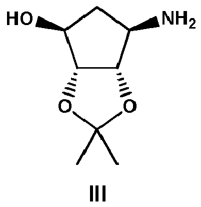 to obtain a compound of formula **VI".**

The compound of formula **II"** can be obtained from the compound of formula **I'** 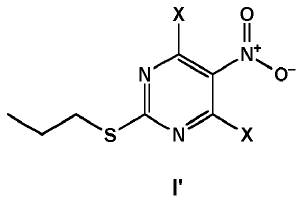 wherein X is Cl or Br,
either by first reducing the nitro group of the compound of formula I' to obtain the compound of formula I", and
subsequently substituting X with PLG by reaction of a reagent PLG-H in the presence of a base, or
by first substituting X with PLG by reaction of a reagent PLG-H in the presence of a base, and subsequently reducing the nitro group of the compound of formula II'.

As set forth above, it is possible and corresponds to a particularly preferred embodiment of the present invention that several steps can be performed through one-pot conversions, without the need of isolation or separation of intermediate compounds. Accordingly, the possibility of reducing the number of required reaction steps, of increasing reaction selectivity, and of simplifying reactions respectively strongly contributes to provide an improved industrially applicable and economically beneficial process for obtaining triazolopyrimidine compounds and specifically ticagrelor. Thus, while of course separation or isolation of any of the intermediate compounds of formulae **IV", VI'** and **VI"** can be carried out to obtain such compounds as useful intermediate compounds, this can be beneficially dispensed with if desired.

For example, methoxylation of heteroaryl halide, N-arylation of **AMAL (III)** and reduction of the nitro group can be carried in one-pot reaction simply by adding reagents and optionally an additional base or solvent.

In a characteristic part of the preferred embodiment, a conversion of **CLINA (I")** to **CLTAM (Va)** can be carried out in one-pot by combining the step of N-arylation of **AMAL (III)** with the nitrosation step by adding excess of acetic acid simultaneously neutralising the present base and preparing the medium suitable for nitrosation.

In another embodiment, a compound of formula VII can be prepared by providing a compound of formula **I'** 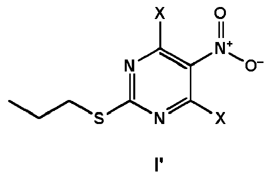 wherein X is Cl or Br,
substituting X with PLG by reaction of a reagent PLG-H in the presence of a base, to obtain a compound of formula **II'** 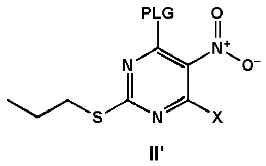 reacting the compound of formula **II'** with a compound of formula **III** 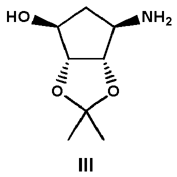 to obtain a compound of formula **VI'** 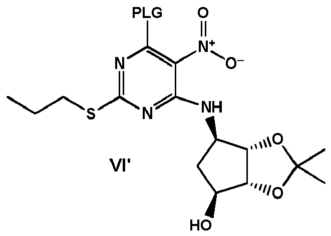 reducing the nitro group of the compound of formula **VI'** to obtain a compound of formula **VI"** 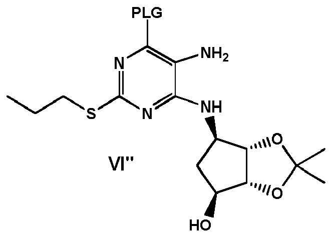 wherein PLG is defined as above, and
converting the compound of formula **VI"** into the compound of formula **VII** by nitrosation,
wherein all said steps are carried out in one pot.

In a characteristic part of the preferred embodiment, a one-pot process for the preparation of **MOTAM (VIIa)** from **CLIN (I')** is carried out. The PLG group is selected from alkoxy, preferably methoxy or benzyloxy. Methoxy substituted derivatives can be simply prepared in alkali metal methoxide solution in methanol, preferably in 1:1 molar methoxide/intermediate ratio. Reagents for other transformations can be taken from prior art approaches on heteroaryl halide analogues, such as iron in acetic acid for reduction of nitro group, reaction with **AMAL** in the presence of base such triethylamine or carbonate, nitrosation with organic nitrite in aprotic solvent or inorganic nitrite in acetic acid. The inertness of PLG enables the reduction of nitro group to be carried out in basic conditions using formamidinesulfinic acid (thiourea dioxide) or sodium dithionite, preferably formamidinesulfinic acid. Therefore, if basic conditions are used, the reduction of nitro group and subsequent nitrosation can be performed without isolating the intermediates, which represents a considerable shortening of the synthesis of substituted benzotriazoles.

A summary of the afore-mentioned one-pot processes is shown in the following scheme 11 below.

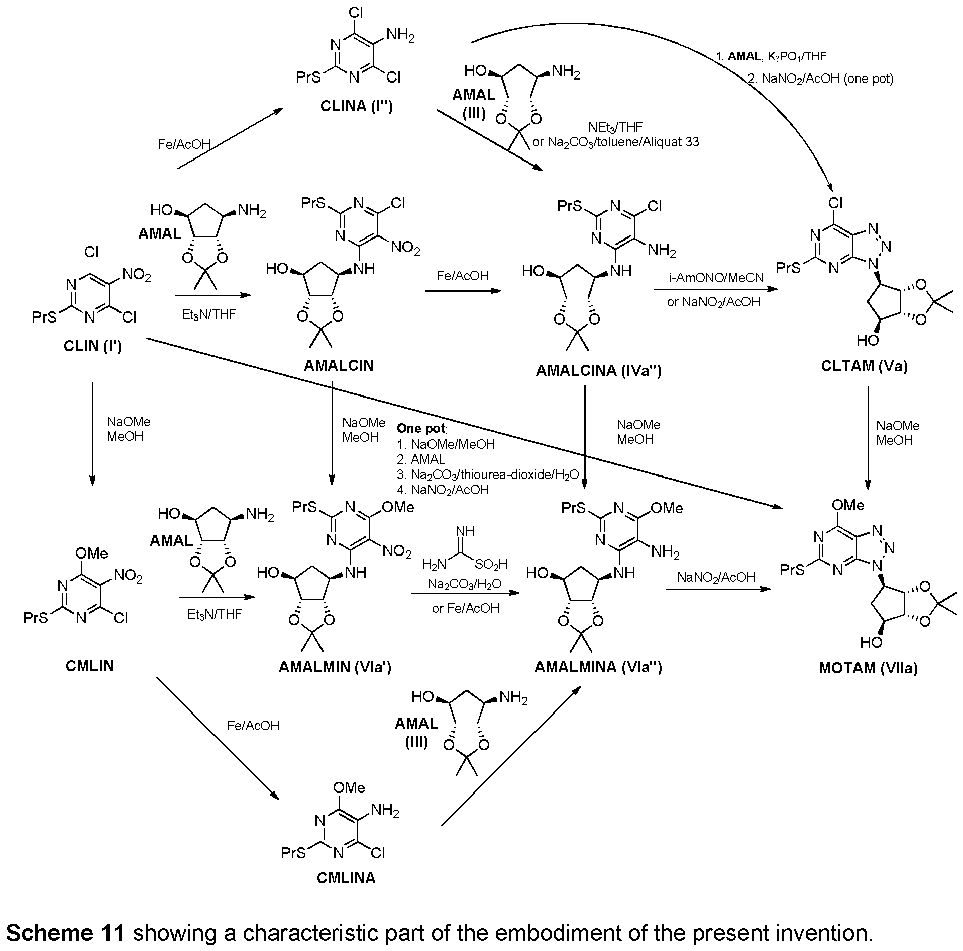

In another embodiment, the introduction of PLG group and the alkylation step can be joined using the same base and solvent (conversion of **CLTAM (Va)** to **VIII).** Furthermore, alkylation and reduction can be carried out in the same reaction mixture (conversion of **VII** to **VIII").** For example in a special case the intermediate **MOTAME (VIIIa')** is not isolated but it can be further transferred to the reduction step.

Using one-pot procedure as described in Scheme 11 and one-pot introduction of hydroxyethyl group reduces the overall procedure of the synthesis of ticagrelor to only four isolation steps starting from 4,6-dichloro-5-nitro-2-(propylthio)pyrimidine **(CLIN = I').**

In a further embodiment, the PLG group can be created indirectly from heteroaryl halides via more accessible intermediates as shown in Scheme 12. Some N-amidyl and N-imidyl groups can be prepared by alkylation and acylation of amino group.

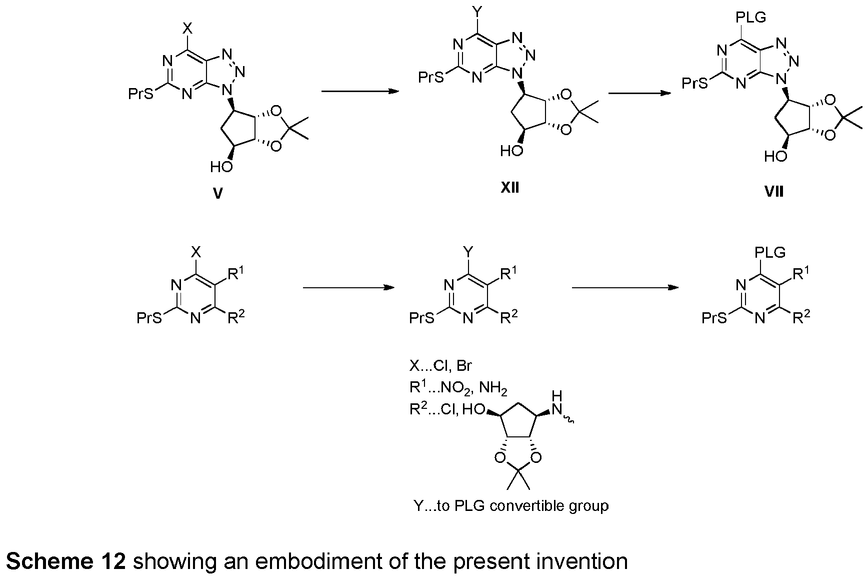

In a further embodiment, the compound of formula **VII** is efficiently O-alkylated, preferably O-alkylated with halides or sulfonates of moieties which can be converted to 2-hydroxyethyl group. Suitable reagents for such conversion are selected from haloacetic or sulfonyloxyacetic esters, preferably C₁-C₄-alkyl bromoacetates, most preferably methyl bromoacetate. Reaction of alkylation is performed in the presence of a strong base preferably sodium hydride to give intermediate **VIII'**. The ester functionality of compound **VIII'** is further reduced by use of hydrides, selected from aluminium or boron hydrides preferebly from lithium aluminium hydride or alkali metal or zinc borohydride, most preferably from lithium borohydride.

The obtained compound of formula **VIII"** is transformed to the protected P2Y12 receptor antagonist preferably to propylidene derivative **CPATAMA** (**X**). Substitution of PLG group with amine side chain, preferably **CPA** (**IX**) is carried out either under net conditions (without solvent) or in a solvent, selected from non-nucleophilic solvents, preferably it is selected form nitriles, ethers, sulphoxides, sulfones, amides or a mixture thereof, more preferably from sulfoxides and amide solvents such as N-alkyl substituted acetamides, pyrrolidones and ureas, most preferably from dimethylsulfoxide and N,N-dimethylacetamide at temperatures from -20 to 100 °C, most preferably at slightly elevated temperature from 25 to 70°C. Thus, despite lower reactivity of PLG, comparing to halogens substitution, the reaction surprisingly does not need harsh conditions. In the case of PLG being selected among the alkoxy groups derived from alcohols liquid at the reaction temperatures, these corresponding alcohols can also be efficiently used as solvents (*e.g*., methanol can be used when PLG is the methoxy group).

In a characteristic example of the embodiment, the methoxy intermediate **MOTAME (VIII')** is reduced by lithium borohydride to give **MOTAMA (VIII"),** which is further carried out to the nucleophilic aromatic substitution with cyclopropyl amine **CPA (IX),** where the methoxy group is in a role of a leaving group. Although in many heteroaryl systems methoxy group is not a satisfactory leaving group it was surprisingly found that the substitution in the triazolopyrimidine system performs smoothly with good yields. In opposite, the group is inert to oxy nucleophiles which can be formed during the alkylation step and no essential amounts of dimeric and polymeric side products were found when the reaction is conducted bellow -10°C.

In an alternative embodiment of the invention, PLG is substituted in the intermediate step of introduction of hydroxyethyl group. Thus, the intermediate **VIII'** is first treated with **CPA** (**IX**) in analogous conditions to those described for amination of compound VIII", to generate the intermediate **XIII,** which is further reduced to give **CPATAMA (X).** Both options are represented in Scheme 13.

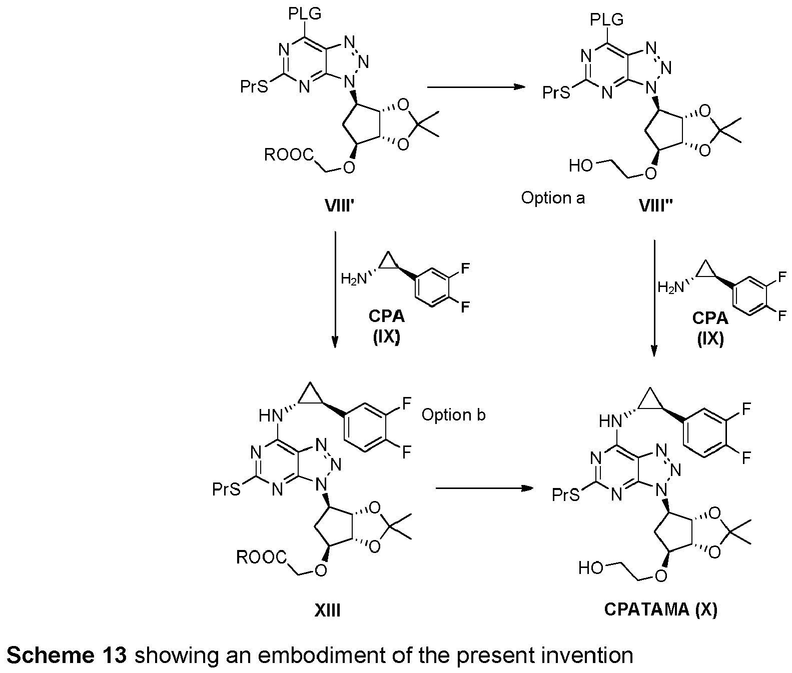

Preparation of ticagrelor (**TCG, XI**) with the formula shown below follows prior art knowledge, wherein 2,2-propylidene protection group of glycolic part of cyclopentane is removed by using acids in protic solvents such as strong acids in alcohols or water or mixtures thereof, preferably hydrochloric or phosphoric acid in methanol or ethanol. If desired, a salt or a co-crystal of the compound of ticagrelor can be optionally prepared.

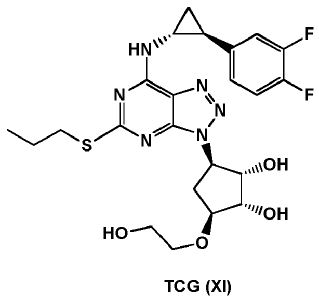

The ticagrelor compound prepared according to the invention may be used or administered on its own, preferably it is administered as a pharmaceutical composition comprising ticagrelor and a pharmaceutically acceptable excipient and/or carrier. Further, the ticagrelor compound prepared according to the invention may be combined with other drugs, especially drugs having activity against platelet aggregation or thrombolytic events.

In a further aspect of the present invention, a pharmaceutical composition comprising the compound of formula **XI** (ticagrelor, **TCG**), a salt or a co-crystal thereof is prepared by comprising the steps of preparing the compound of formula **XI,** a salt or a co-crystal thereof as described above, and mixing the compound of formula **XI,** a salt or a co-crystal thereof with a pharmaceutically acceptable carrier and/or excipient. The administration form can be suitably chosen, e.g. a form suitable for oral, parenteral, rectal administration and/or administration by inhalation, and the dosage form may be solid, liquid, or powdery. Therefore, the pharmaceutical composition comprising ticagrelor compound prepared according to the invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the compound or a finely divided form thereof, or particles comprising the compound, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The compound or a finely divided form thereof or particles containing the same may be coated by another substance. The powder mixture or particles containing the compound may also be dispensed into capsules.

The pharmaceutical composition comprising ticagrelor prepared according to the invention in a desired dose is generally suitable to treat a disease or condition of a patient in need thereof, specifically to display a desired activity against platelet aggregation, or in the treatment or prophylaxis of thrombolytic events.

Further aspects of the present invention reside in the provision of valuable intermediate compounds **II, VI** and **VIII** useful in the synthesis of a compound of ticagrelor (**TCG, XI**), which intermediate compounds respectively have in common the protecting-leaving group PLG: 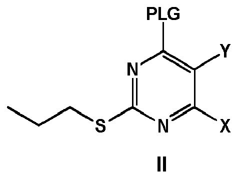 wherein PLG is a protecting-leaving group, X is Cl or Br, and Y is NO₂ or NH₂, 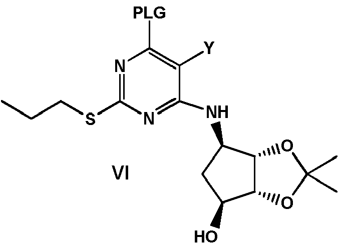 wherein PLG is a protecting-leaving group, and Y is NO₂ or NH₂, 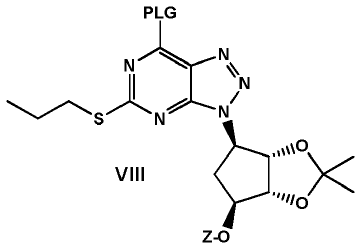 wherein PLG is a protecting-leaving group, and Z is hydrogen, hydroxyethyl or a group convertible to hydroxyethyl.

As to the definition of "PLG" and "Z", reference is made to the descriptions elsewhere in the present specification.

Particular examples of such useful intermediate compounds are listed by their respective formulas below (in these formulas, "Pr" denotes "n-propyl"):

| **Formula** | **Chemical name** |
|---|---|
| 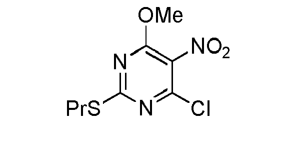 | 4-chloro-6-methoxy-5-nitro-2-(propylthio)pyrimidine |
| 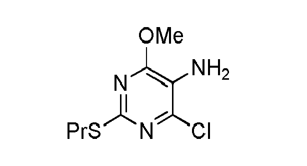 | 4-chloro-6-methoxy-2-(propylthio)pyrimidin-5-amine |
| 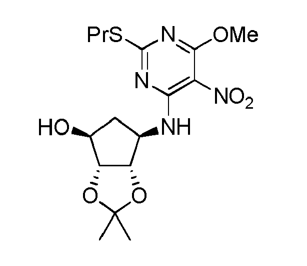 | (3a*R*,4*S*,6*R*,6a*S*)-6-((6-methoxy-5-nitro-2-(propylthio)pyrimidin-4-yl)amino)-2,2-dimethyltetrahydro-3aH-cyclopenta[d][1,3]dioxol-4-ol |
| 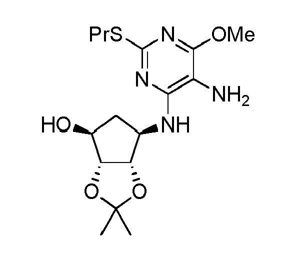 | (3a*R*,4*S*,6*R*,6a*S*)-6-((5-amino-6-methoxy-2-(propylthio)pyrimidin-4-yl)amino)-2,2-dimethyltetrahydro-3a*H*-cyclopenta[*d*][1,3]dioxol-4-ol |
| 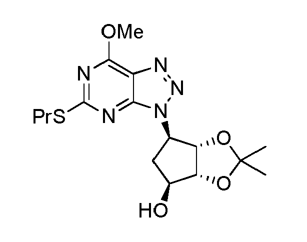 | (3a*R*,4*S*,6*R*,6a*S*)-6-(7-Methoxy-5-(propylthio)-3*H*-[1,2,3]triazolo[4,5-*d*]pyrimidin-3-yl)-2,2-dimethyltetrahydro-3aH-cyclopenta[*d*][1,3]dioxol-4-ol |
| 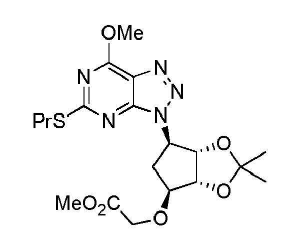 | Methyl 2-(((3a*R*,4*S*,6*R*,6a*S*)-6-(7-methoxy-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-yl)oxy)acetate |
| 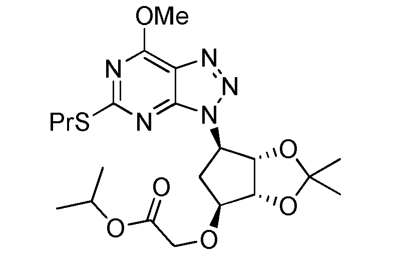 | Isopropyl 2-(((3a*R*,4*S*,6*R*,6a*S*)-6-(7-methoxy-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-yl)oxy)acetate |
| 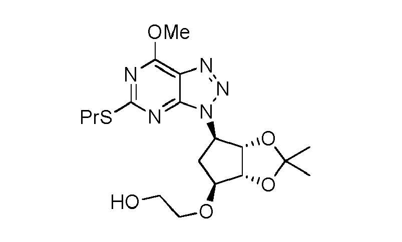 | 2-(((3a*R*,4*S*,6*R*,6a*S*)-6-(7-Methoxy-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-yl)oxy)ethanol |
| 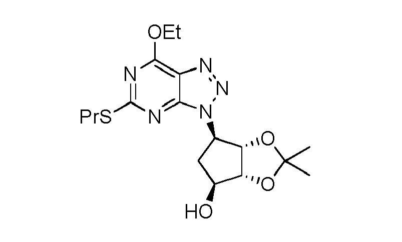 | (3a*R*,4*S*,6*R*,6a*S*)-6-(7-ethoxy-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-ol |
| 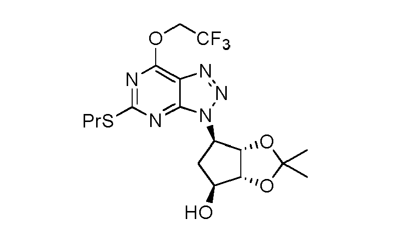 | (3a*R*,4*S*,6*R*,6a*S*)-2,2-dimethyl-6-(5-(propylthio)-7-(2,2,2-trifluoroethoxy)-3*H*-[1,2,3]triazolo[4,5-*d*]pyrimidin-3-yl)tetrahydro-3a*H*-cyclopenta[*d*][1,3]dioxol-4-ol |
| 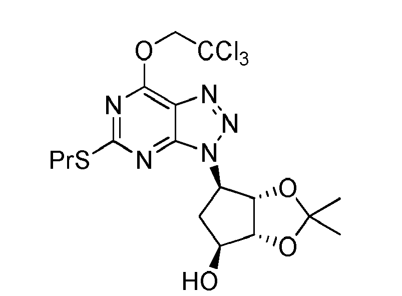 | (3a*R*,4*S*,6*R*,6a*S*)-2,2-dimethyl-6-(5-(propylthio)-7-(2,2,2-trichloroethoxy)-3*H*-[1,2,3]triazolo[4,5-*d*]pyrimidin-3-yl)tetrahydro-3a*H*-cyclopenta[*d*][1,3]dioxol-4-ol |
| 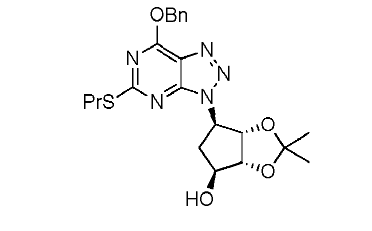 | (3a*R*,4*S*,6*R*,6a*S*)-6-(7-(benzyloxy)-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-ol |
| 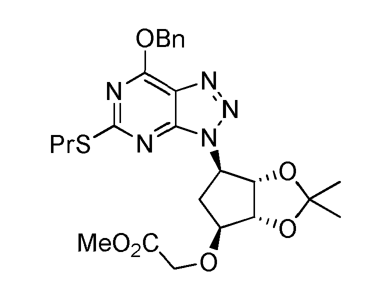 | methyl 2-(((3a*R*,4*S*,6*R*,6a*S*)-6-(7-(benzyloxy)-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-yl)oxy)acetate |
| 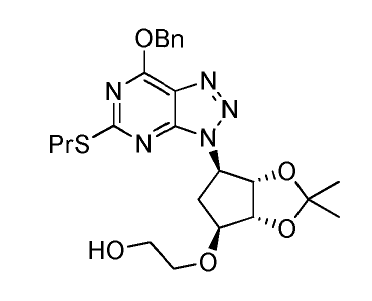 | 2-(((3a*R*,4*S*,6*R*,6a*S*)-6-(7-(benzyloxy)-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-yl)oxy)ethanol |
| 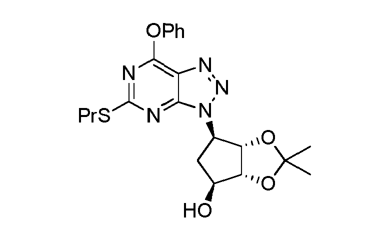 | (3a*R*,4*S*,6*R*,6a*S*)-2,2-Dimethyl-6-(7-phenoxy-5-(propylthio)-3*H*-[1,2,3]triazolo[4,5-*d*]pyrimidin-3-yl)tetrahydro-3a*H*-cyclopenta[*d*][1,3]dioxol-4-ol |
| 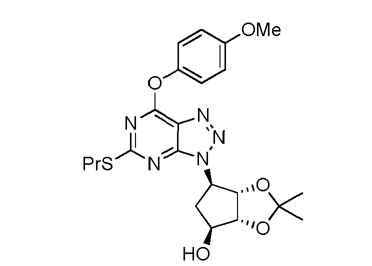 | (3a*R*,4*S*,6*R*,6a*S*)-6-(7-(4-methoxyphenoxy)-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-ol |
| 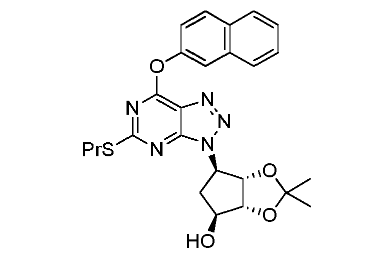 | (3a*R*,4*S*,6*R*,6a*S*)-2,2-dimethyl-6-(7-(naphthalen-2-yloxy)-5-(propylthio)-3*H*-[1,2,3]triazolo[4,5-*d*]pyrimidin-3-yl)tetrahydro-3a*H*-cyclopenta[*d*][1,3]dioxol-4-ol |
| 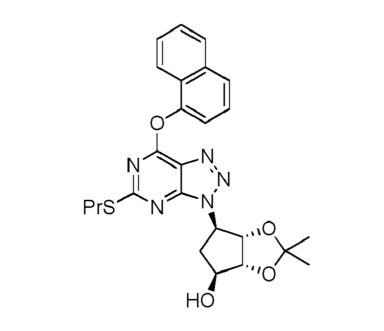 | (3a*R*,4*S*,6*R*,6a*S*)-2,2-dimethyl-6-(7-(naphthalen-1-yloxy)-5-(propylthio)-3*H*-[1,2,3]triazolo[4,5-*d*]pyrimidin-3-yl)tetrahydro-3a*H*-cyclopenta[*d*][1,3]dioxol-4-ol |
| 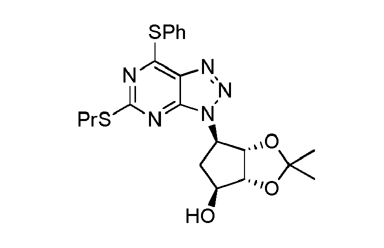 | (3a*R*,4*S*,6*R*,6a*S*)-2,2-dimethyl-6-(7-(phenylthio)-5-(propylthio)-3*H*-[1,2,3]triazolo[4,5-*d*]pyrimidin-3-yl)tetrahydro-3a*H*-cyclopenta[*d*][1,3]dioxol-4-ol |
| 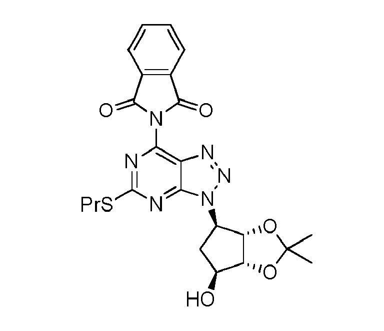 | 2-(3-((3a*S*,4*R*,6*S*,6a*R*)-6-hydroxy-2,2-dimethyltetrahydro-3a*H*-cyclopenta[*d*][1,3]dioxol-4-yl)-5-(propylthio)-3*H*-[1,2,3]triazolo[4,5-*d*]pyrimidin-7-yl)isoindoline-1,3-dione |
| 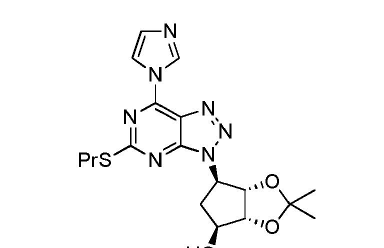 HU | (3a*R*,4*S*,6*R*,6a*S*)-6-(7-(1*H*-Imidazol-1-yl)-5-(propylthio)-3*H*-[1,2,3]triazolo[4,5-*d*]pyrimidin-3-yl)-2,2-dimethyltetrahydro-3a*H*-cyclopenta[*d*][1,3]dioxol-4-ol |

In the following the present invention will be described in further detail by illustrative, nonlimiting examples.

### Experimental Procedures

### Example 1: Preparation of 4,6-dichloro-2-(propylthio)pyrimidin-5-amine (CLINA)

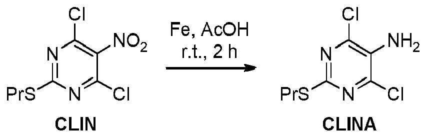

To a mixture of Fe (167 g, 3 mol) in AcOH (1 L) 4,6-dichloro-5-nitro-2-(propylthio)pyrimidine (**CLIN**, 100 g, 0.37 mol) was slowly added over 2 h, and reaction mixture was then stirred at room temperature for additional 2 h. Salts were then filtered off and the filtrate concentrated. EtOAc was added (400 mL), organic layer was washed with water (3 x 200 mL), dried over MgSO₄, and concentrated to afford oily product (65.2 g, 73% yield). ¹H NMR (CDCl₃, 500 MHz) δ 1.03 (t, *J* = 7.4 Hz, 3H), 1.73 (m, 2H), 3.07 (m, 2H), 4.23 (br s, 2H); MS (ESI) *m*/*z*: 238 [MH]⁺.

### Example 2: Preparation of (3aR,4S,6R,6aS)-6-((6-chloro-5-nitro-2-(propylthio)pyrimidin-4-yl)amino)-2,2-dimethyltetrahydro-3aH-cyclopenta[d][1,3]dioxol-4-ol (AMALCIN)

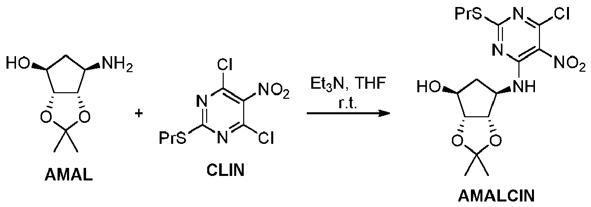

The title compound was prepared using the method described in WO 00/34283.

To a solution of **CLIN** (11.6 g, 43.3 mmol) and Et₃N (4.0 mL, 28.9 mmol) in dry THF (100 mL) solution of **AMAL** (5.0 g, 28.9 mmol) in dry THF (100 mL) was slowly added at room temperature, and resulting reaction mixture was stirred for 1 h. Salts were filtered off, washed with dry THF (50 mL), filtrate was concentrated, and crude product was purified by crystallization from hexane/EtOAc mixture to afford yellowish powder (m = 9.88 g, 84% yield). MP 63°C; ¹H NMR (DMSO-d₆, 500 MHz) δ 0.98 (t, *J* = 7.3 Hz, 3H), 1.19 (s, 3H), 1.34 (s, 3H), 1.64-1.78 (m, 3H), 2.17 (m, 1H), 3.04 (m, 1H), 3.12 (m, 1H), 4.11 (m, 1H), 4.44 (m, 1H), 4.53-4.58 (m, 2H), 8.76 (br d, *J* = 7.9 Hz, 1H); MS (ESI) *m*/*z*: 405 [MH]⁺.

### Example 3: Preparation of (3aR,4S,6R,6aS)-6-((5-amino-6-chloro-2-(propylthio)pyrimidin-4-yl)amino)-2,2-dimethyltetrahydro-3aH-cyclopenta[d][1,3]dioxol-4-ol (AMALCINA)

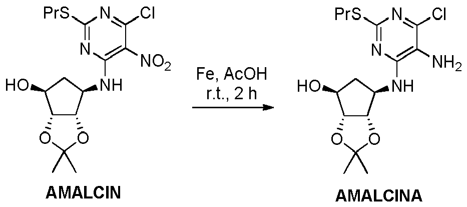

The title compound was prepared using the method described in WO 00/34283.

A solution of **AMALCIN** (0.50 g, 1.23 mmol) was slowly added to a stirring mixture of AcOH (3 mL) and Fe (0.84 g, 15.0 mmol). Resulting reaction mixture was stirred at room temperature for 2 h. Then salts were filtered off, and AcOH evaporated. Water was added (20 mL), and product was extracted to EtOAc (3 x 10 mL). Combined organic layers were washed with saturated Na₂CO₃ (3 x 10 mL), dried over MgSO₄, and concentrated to afford title compound as brown syrup (m = 0.44 g, 96% yield). ¹H NMR (CDCl₃, 500 MHz) δ 0.96 (t, *J* = 7.3 Hz, 3H), 1.18 (s, 3H), 1.37 (s, 3H), 1.68 (m, 2H), 1.78 (m, 1H), 2.27 (m, 1H), 2.94 (m, 1H), 3.08 (m, 1H), 3.30 (br s, 2H), 4.34 (m, 1H), 4.46-4.55 (m, 4H), 6.12 (br d, *J* = 8.3 Hz, 1H); MS (ESI) *m*/*z*: 375 [MH]⁺.

**AMALCINA** was also prepared from **AMAL** and **CLINA.**

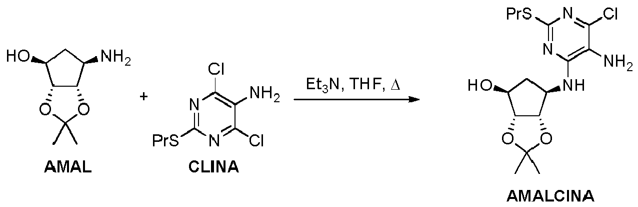

To a solution of **AMAL** (7.64 g, 44.1 mmol) and **CLINA** (10.5 g, 44.1 mmol) in dry THF (40 mL) Et₃N (6.76 mL, 48.5 mmol) was added at room temperature. Resulting reaction mixture was stirred at reflux for 24 h, then salts were filtered off, and solvent evaporated to afford crude product, which was then purified by chromatography (SiO₂, hexane:EtOAc). Brown syrup (14.4 g, 87% yield).
Or

To a solution of **AMAL** (1.45 g, 8.40 mmol) and **CLINA** (2.0 g, 8.40 mmol) in toluene (20 mL) Na₂CO₃ (1.07 g, 10.1 mmol) and Aliquat 336 (0.34 g, 0.84 mmol) were added at room temperature. Resulting reaction mixture was stirred at 100°C for 48 h, then salts were filtered off, and solvent evaporated to afford crude product, which was then purified by chromatography (SiO₂, hexane: EtOAc). Brown syrup (2.52 g, 80% yield).

### Example 4: Preparation of (3aR,4S,6R,6aS)-6-(7-chloro-5-(propylthio)-3H-[1,2,3]triazolo[4,5-d]pyrimidin-3-yl)-2,2-dimethyltetrahydro-3aH-cyclopenta[d][1,3]dioxol-4-ol (CLTAM)

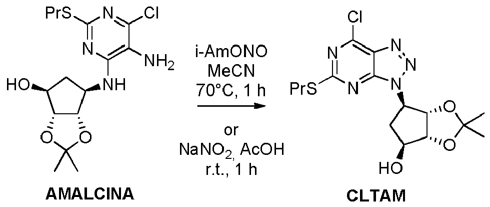

The title compound was prepared according the method described in WO 00/34283

A solution of **AMALCINA** (6.0 g, 16.0 mmol) and i-AmONO (3.23 mL, 24.0 mmol) in dry MeCN (100 mL) was stirred at 70°C for 1 h, then solvent was evaporated to afford crude product, which was then purified by chromatography (SiO₂, hexane:EtOAc). Slightly yellowish oil which crystalized upon standing. (5.70 g, 92% yield). Mp 83°C; ¹H NMR (CDCl₃, 500 MHz) δ 1.09 (t, *J* = 7.4 Hz, 3H), 1.32 (s, 3H), 1.53 (s, 3H), 1.82 (m, 2H), 2.39 (m, 1H), 2.88 (m, 1H), 3.22 (m, 2H), 3.78 (d, *J* = 8.4 Hz, 1H), 4.43 (m, 1H), 4.78 (m, 1H), 5.07 (m, 1H), 5.32 (m, 1H); MS (ESI) *m*/*z*: 386 [MH]⁺.

The title compound was also prepared using the method described in WO 01/92263.

To a solution of **AMALCINA** (1.0 g, 2.67 mmol) in AcOH (5 mL) at room temperature NaNO₂ (0.20 g, 2.64 mmol) was slowly added. Resulting reaction mixture was stirred at room temperature for 1 h, then AcOH was evaporated, water (50 mL) was added, and product was extracted to MeTHF (3 x 10 mL). Combined organic phases were washed with saturated Na₂CO₃ (3x10 mL), dried over MgSO₄, and concentrated to afford crude product, which was then crystalized from hexane/EtOAc mixture. White powder (0.95 g, 92% yield).

**CLTAM** was also prepared through one-pot reaction starting from **AMAL** and **CLINA**.

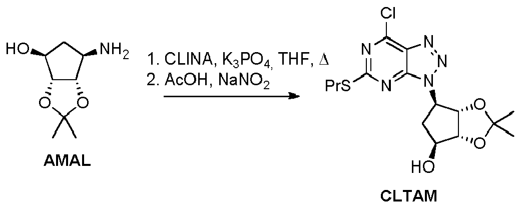

To a solution of **AMAL** (7.64 g, 44.1 mmol) and **CLINA** (10.5 g, 44.1 mmol) in dry THF (40 mL) K₃PO₄ (10.3 g, 48.5 mmol) was added at room temperature. Resulting reaction mixture was stirred at reflux for 24 h, then AcOH was slowly added (100 mL) followed by NaNO₂ (3.65 g, 52.9 mmol). Reaction mixture was stirred at room temperature por 1 h, then solvents were evaporated, water (100 mL) was added, and product was extracted to MeTHF (3 x 50 mL). Combined organic phases were washed with saturated NaHCO₃ (3 x 50 mL), dried over MgSO₄, and concentrated to afford crude product, which was then purified by chromatography (SiO₂, hexane:EtOAc) to afford colorless crystals (12.2 g, 72% yield).

### Example 5: Preparation of 4-chloro-6-methoxy-5-nitro-2-(propylthio)pyrimidine (CMLIN)

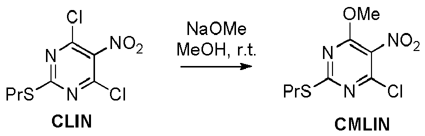

To a solution of **CLIN** (1.0 g, 3.73 mmol) in dry MeOH (10 mL) NaOMe (0.20 g, 3.73 mmol) was slowly added, and reaction mixture was then stirred at room temperature for 1 h. AcOH (1 mL) and water (20 mL) were added, and product was extracted to MeTHF (3 x 10 mL). Combined organic phases were dried over MgSO₄ and concentrated to afford crude product, which was then purified by chromatography (SiO₂, hexane:EtOAc) to afford yellow oil (0.78 g, 79% yield). MS (ESI) *m*/*z*: 264 [MH]⁺.

### Example 6: Preparation of 4-chloro-6-methoxy-2-(propylthio)pyrimidin-5-amine (CMLINA)

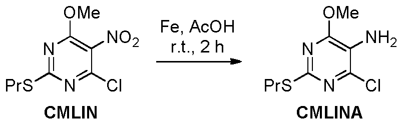

To a solution of **CMLIN** (0.10 g, 0.57 mmol) in AcOH (2 mL) Fe (98 mg, 1.75 mmol) was added, and reaction mixture was then stirred at room temperature for 2 h. Salts were then filtered off and filtrate concentrated. EtOAc was added (400 mL), organic layer was washed with water (3 x 200 mL), dried over MgSO₄, and concentrated to afford oily product (0.60 g, 68% yield). MS (ESI) *m*/*z*: 234 [MH]⁺.

### Example 7: Preparation of (3aR,4S,6R,6aS)-6-((6-methoxy-5-nitro-2-(propylthio)pyrimidin-4-yl)amino)-2,2-dimethyltetrahydro-3aH-cyclopenta[d][1,3]dioxol-4-ol (AMALMIN)

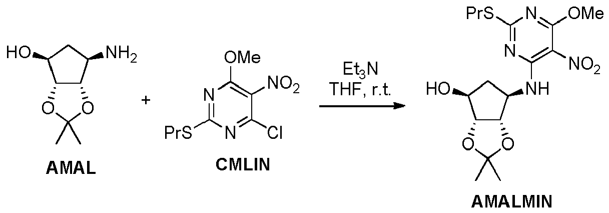

To a solution of **CMLIN** (0.15 g, 0.57 mmol) and Et₃N (87 µL, 0.63 mmol) in dry THF (2 mL) **AMAL** (0.10 g, 0.57 mmol) was slowly added, and reaction mixture was then stirred at room temperature for 1 h. Salts were filtered off and filtrate was concentrated to afford yellow oil (0.22 g, 96% yield). MS (ESI) *m*/*z*: 401 [MH]⁺.

**AMALMIN** was also prepared from **AMALCIN.**

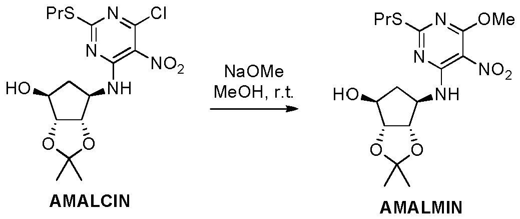

To a solution of **AMALCIN** (1.78 g, 4.40 mmol) in dry MeOH (10 mL) NaOMe (0.25 g, 4.62 mmol) was slowly added, and reaction mixture was then stirred at room temperature for 1 h. AcOH (1 mL) and water (50 mL) were added, and product was extracted to CH₂Cl₂ (3 x 20 mL). Combined organic phases were dried over MgSO₄ and concentrated to afford crude product, which was then purified by chromatography (SiO₂, hexane:EtOAc) to afford yellowish powder (1.49 g, 85% yield).

### Example 8: Preparation of (3aR,4S,6R,6aS)-6-((5-amino-6-methoxy-2-(propylthio)pyrimidin-4-yl)amino)-2,2-dimethyltetrahydro-3aH-cyclopenta[d][1,3]dioxol-4-ol (AMALMINA)

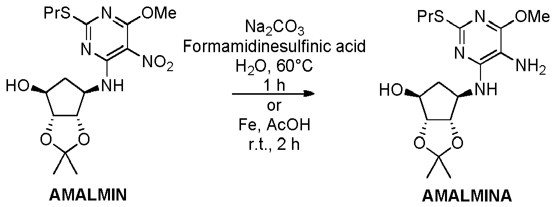

A mixture of **AMALMIN** (0.40 g, 1.0 mmol), Na₂CO₃ (0.35 g, 3.3 mmol) and formamidinesulfinic acid (0.36 g, 3.3 mmol) in MeOH (5 mL) and water (0.5 mL) was stirred at 60°C for 1 h. Then water was added (20 mL), and product was extracted to MeTHF (3x10 mL). Combined organic layers were dried over MgSO₄, and concentrated to afford crude product, which was then purified by chromatography (SiO₂, hexane:EtOAc) to give **AMALMINA** as brown oil (m = 0.31 g, 85% yield). MS (ESI) *m*/*z*: 371 [MH]⁺.

The title compound was prepared using the method described in WO 00/34283.

A solution of **AMALMIN** (1.0 g, 2.50 mmol) was slowly added to a stirring mixture of AcOH (20 mL) and Fe (1.40 g, 25.0 mmol). Resulting reaction mixture was stirred at room temperature for 2 h. Then salts were filtered off, and AcOH evaporated. Water was added (20 mL), and product was extracted to MeTHF (3 x 10 mL). Combined organic layers were washed with saturated Na₂CO₃ (3 x 10 mL), dried over MgSO₄, and concentrated to afford title compound as brown syrup (m = 0.74 g, 80% yield).

### Example 9: Preparation of (3aR,4S,6R,6aS)-6-(7-methoxy-5-(propylthio)-3H-[1,2,3]triazolo[4,5-d]pyrimidin-3-yl)-2,2-dimethyltetrahydro-3aH-cyclopenta[d][1,3]dioxol-4-ol (MOTAM)

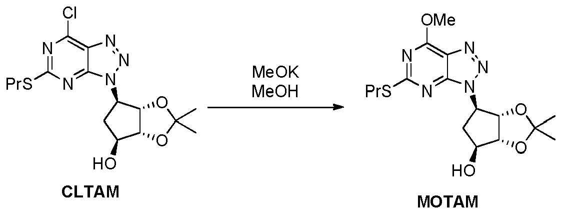

To a solution of **CLTAM** (0.50 g, 1.30 mmol) in dry MeOH (2 mL) at room temperature 25% solution of KOMe in MeOH (0.42 mL, 1.43 mmol) was added, and resulting reaction mixture was stirred for 15 min. Water was added (10 mL), and product was extracted to MeTHF (3 x 5 mL). Combined organic layers were dried over MgSO₄, and concentrated to afford title compound as brownish syrup (m = 0.45 g, 90% yield). ¹H NMR (CDCl₃, 500 MHz) δ 1.05 (t, *J* = 7.3 Hz, 3H), 1.28 (s, 3H), 1.49 (s, 3H), 1.79 (m, 2H), 2.29 (m, 1 H), 2.85 (m, 1H), 2.56 (m, 1H), 3.17 (m, 2H), 4.20 (s, 3H), 4.38 (br d, *J* = 5.2 Hz, 1H), 4.46 (br s, 1H), 4.76 (m, 1H), 4.95 (m, 1H), 5.30 (m, 1H); MS (ESI) *m*/*z*: 382 [MH]⁺.

**MOTAM** was also prepared from **CLTAM** using K₂CO₃ / MeOH.

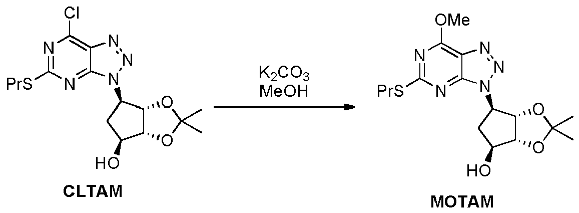

To a solution of **CLTAM** (10.0 g, 25.9 mmol) in dry MeOH (50 mL) at room temperature K₂CO₃ (3.94 g, 28.5 mmol) was added, and resulting reaction mixture was stirred for 2 h. Solvent was evaporated, MeTHF (100 mL) was added, and salts were filtered off. Organic phase was washed with brine (3 x 100 mL) and water (2 x 100 mL), dried over MgSO₄, and concentrated to afford title compound as yellowish syrup (m = 9.39 g, 95% yield).

**MOTAM** was also prepared from **AMALMINA.**

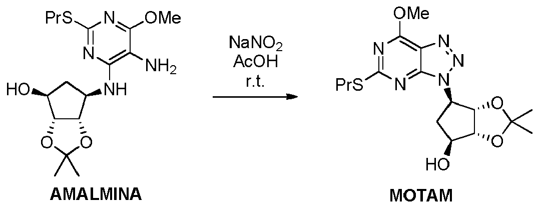

To a solution of **AMALMINA** (0.5 g, 1.35 mmol) in AcOH (5 mL) at room temperature NaNO₂ (0.10 g, 1.48 mmol) was added. Resulting reaction mixture was stirred at room temperature for 1 h, then AcOH was evaporated, water (50 mL) was added, and product was extracted to MeTHF (3x10 mL). Combined organic phases were washed with saturated NaHCO₃ (3x10 mL), dried over MgSO₄, and concentrated to afford title compound as colorless oil (0.46 g, 90% yield).

**MOTAM** was also prepared through one-pot reaction starting from **CLIN.**

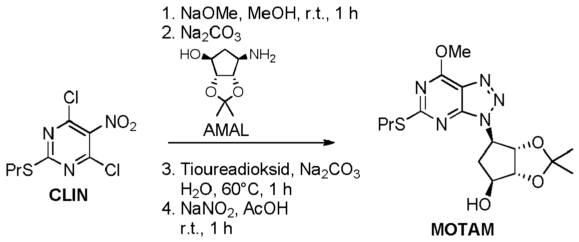

To a solution of **CLIN** (0.27 g, 1.0 mmol) in dry MeOH (5 mL) NaOMe (54 mg, 1.0 mmol) was added, and reaction mixture was then stirred at room temperature for 1 h. Then Et₃N (0.15 mL, 1.1 mmol) and **AMAL** (0.18 g, 1.05 mmol) were added, and reaction mixture was stirred at room temperature for 1 h. Then Na₂CO₃ (0.35 g, 3.3 mmol), formamidinesulfinic acid (0.36 g, 3.3 mmol) and water (0.5 mL) were added. Resulting reaction mixture was stirred at 60°C for 1 h, then AcOH (10 mL) was slowly added, followed by NaNO₂ (76 mg, 1.1 mmol). Resulting reaction mixture was stirred at room temperature for 1 h, then volatile components were evaporated, water (40 mL) was added, and product was extracted to MeTHF (3 x 10 mL). Combined organic phases were washed with saturated NaHCO₃ (3x10 mL), dried over MgSO₄, and concentrated to afford crude product, which was purified by chromatography (SiO₂, hexane:EtOAc) to give **MOTAM** as colorless oil (0.20 g, 52% yield).

### Example 10: Preparation of (3aR,4S,6R,6aS)-2,2-dimethyl-6-(5-(propylthio)-7-(2,2,2-trifluoroethoxy)-3H-[1,2,3]triazolo[4,5-d]pyrimidin-3-yl)tetrahydro-3aH-cyclopenta[d][1,3]dioxol-4-ol (FETAM)

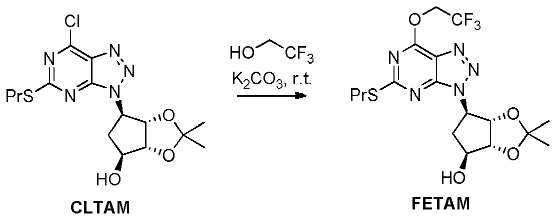

A mixture of **CLTAM** (1.0 g, 2.59 mmol) and K₂CO₃ (0.39 g, 2.85 mmol) in 2,2,2-trifluoroethanol (3 mL) was stirred at room temperature for 1 hour. The solvent was evaporated, MeTHF (10 mL) was added, salts were filtered off, and filtrate was concentrated to afford crude product, which was then recrystallized from CH₂Cl₂/hexane mixture to give title compound as off white powder (1.01 g, 87% yield). MP 107°C; MS (ESI) *m*/*z*: 450 [MH]⁺.

### Example 11: Preparation of (3aR,4S,6R,6aS)-2,2-dimethyl-6-(5-(propylthio)-7-(2,2,2-trichloroethoxy)-3H-[1,2,3]triazolo[4,5-d]pyrimidin-3-yl)tetrahydro-3aH-cyclopenta[d][1,3]dioxol-4-ol (CETAM)

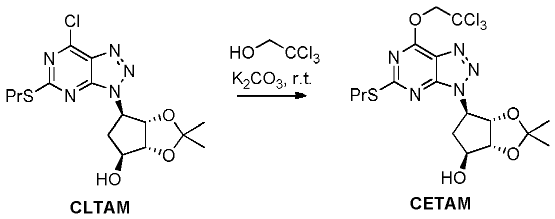

A mixture of **CLTAM** (1.0 g, 2.59 mmol) and K₂CO₃ (0.39 g, 2.85 mmol) in 2,2,2-trichloroethanol (5 mL) was stirred at room temperature for 16 hours. Water (70 mL) was added, then product was extracted to MeTHF (3 x 20 mL), combined organic layers ware dried over MgSO₄, and then concentrated to afford crude product, which was purified by chromatography (SiO₂, hexane:EtOAc) to afford title compound as white powder (1.00 g, 78% yield). MP 118°C; MS (ESI) *m*/*z*: 498 [MH]⁺.

### Example 12: Preparation of (3aR,4S,6R,6aS)-6-(7-ethoxy-5-(propylthio)-3H-[1,2,3]triazolo[4,5-d]pyrimidin-3-yl)-2,2-dimethyltetrahydro-3aH-cyclopenta[d][1,3]dioxol-4-ol (EOTAM)

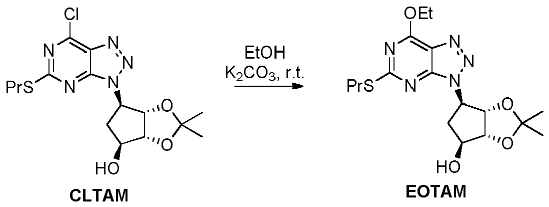

A mixture of **CLTAM** (1.0 g, 2.59 mmol) and K₂CO₃ (0.39 g, 2.85 mmol) in ethanol (5 mL) was stirred at room temperature for 16 hours. The solvent was evaporated, *i*Pr₂O (10 mL) was added, salts were filtered off, and filtrate was concentrated to afford crude product, which was purified by chromatography (SiO₂, hexane:EtOAc) to afford title compound as colorless oil (1.00 g, 98% yield). MS (ESI) *m*/*z*: 396 [MH]⁺.

### Example 13: Preparation of (3aR,4S,6R,6aS)-6-(7-(benzyloxy)-5-(propylthio)-3H-[1,2,3]triazolo[4,5-d]pyrimidin-3-yl)-2,2-dimethyltetrahydro-3aH-cyclopenta[d][1,3]dioxol-4-ol (BOTAM)

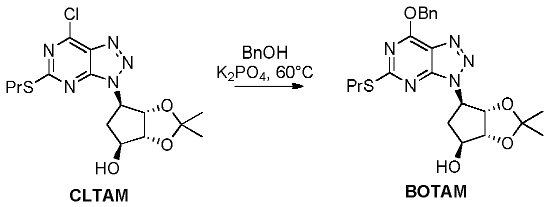

A mixture of **CLTAM** (2.0 g, 5.18 mmol), K₃PO₄ (1.21 g, 5.70 mmol) in benzyl alcohol (10 mL) was stirred at 70°C for 88 hours. AcOH (2 mL) and water (50 mL) were added, then product was extracted to MeTHF (3 x 20 mL). Combined organic layers ware dried over MgSO₄, and then concentrated to afford crude product, which was purified by chromatography (SiO₂, hexane:EtOAc) to afford title compound as white powder (1.71 g, 72% yield). MP 97°C; MS (ESI) *m*/*z*: 458 [MH]⁺.

### Example 14: Preparation of (3aR,4S,6R,6aS)-2,2-dimethyl-6-(7-phenoxy-5-(propylthio)-3H-[1,2,3]triazolo[4,5-d]pyrimidin-3-yl)tetrahydro-3aH-cyclopenta[d][1,3]dioxol-4-ol (FOTAM)

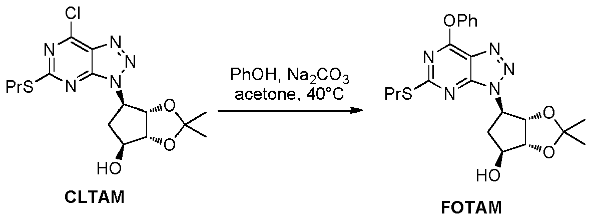

A mixture of **CLTAM** (1.76 g, 4.56 mmol), phenol (0.45 g, 4.79 mmol), and Na₂CO₃ (0.51 g, 4.79 mmol) in acetone (10 mL) was stirred at 40°C for 16 hours, then salts were filtered off, and filtrate concentrated. AcOH (3 mL) and water (20 mL) were added, and product was extracted to MeTHF (3 x 10 mL). Combined organic layers ware dried over MgSO₄, and then concentrated to afford crude product, which was purified by chromatography (SiO₂, hexane:EtOAc) to afford title compound as colorless oil (1.69 g, 83% yield). ¹H NMR (CDCl₃, 500 MHz) δ 0.81 (t, *J* = 7.3 Hz, 3H), 1.30 (s, 3H), 1.49 (s, 3H), 1.53 (m, 2H), 2.32 (m, 1H), 2.82-2.89 (m, 3H), 4.36-4.42 (m, 2H), 4.77 (m, 1H), 5.01 (m, 1H), 5.33 (m, 1H), 7.23 (m, 2H), 7.27 (m, 1H), 7.39-7.43 (m, 2H); MS (ESI) *m*/*z*: 444 [MH]⁺.

### Example 15: Preparation of (3aR,4S,6R,6aS)-6-(7-(4-methoxyphenoxy)-5-(propylthio)-3H-[1,2,3]triazolo[4,5-d]pyrimidin-3-yl)-2,2-dimethyltetrahydro-3aH-cyclopenta[d][1,3]dioxol-4-ol (MFOTAM)

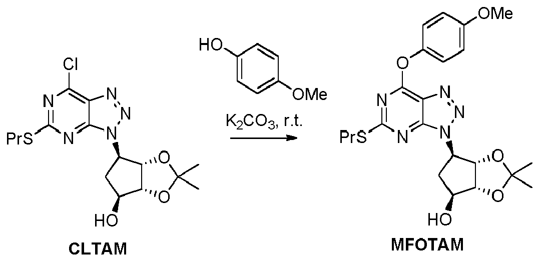

A mixture of **CLTAM** (3.09 g, 8 mmol), *p*-methoxyphenol (1.30 g, 10.5 mmol) and K₂CO₃ (1.44 g, 10.4 mmol) in 2-butanone (40 mL) was stirred 24 h at 25 °C. The reaction mixture was washed with water (2×40 mL) and evaporated under reduced pressure. The residue was recrystallized from a toluene / cyclohexane mixture to give **MFOTAM** as an off-white powder (2.36 g, 62%): 99.2 area% HPLC; mp 114-116 °C; ¹H NMR (CDCl₃, 500 MHz) δ 0.90 (t, *J* = 7.4 Hz, 3H), 1.34 (s, 3H), 1.55 (s, 3H), 1.61 (m, 2H), 2.19 (m, 1H), 2.32 (d, *J* = 15.4 Hz, 1H), 2.90-2.97 (m, 3H), 3.86 (s, 3H), 4.45 (m, 1H), 4.83 (m, 1H), 4.99 (d, *J* = 5.5 Hz, 1H), 5.40 (d, *J* = 8.4 Hz, 1H), 6.97 (AA'XX', 2H), 7.20 (AA'XX', 2H).

### Example 16: Preparation of (3aR,4S,6R,6aS)-2,2-dimethyl-6-(7-(naphthalen-2-yloxy)-5-(propylthio)-3H-[1,2,3]triazolo[4,5-d]pyrimidin-3-yl)tetrahydro-3aH-cyclopenta[d][1,3]dioxol-4-ol (NOTAM)

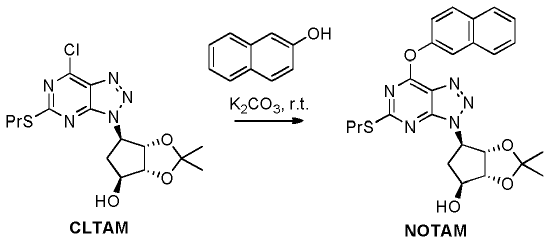

A mixture of **CLTAM** (3.09 g, 8 mmol), 2-naphthol (1.44 g, 10 mmol) and K₂CO₃ (1.44 g, 10.4 mmol) in acetonitrile (40 mL) was stirred 4 h at 25 °C. The reaction mixture was diluted with water (200 mL), extracted with ethyl acetate (80 mL), the extract washed with water (100 mL) and evaporated under reduced pressure to give a crude solid product, which was triturated in warm cyclohexane (50 mL) and filtered. The product **NOTAM** was obtained as a pinkish powder (3.42 g, 87%): 98.9 area% HPLC; mp 147-149 °C; ¹H NMR (CDCl₃, 500 MHz) δ 0.70 (t, 3H, *J* = 7.4 Hz), 1.36 (s, 3H), 1.53 (m, 2H), 1.56 (s, 3H), 2.35 (d, *J* = 15.4 Hz, 1H), 2.86 (t, *J* = 7.5 Hz, 2H), 2.96 (m, 1H), 4.45 (m, 2H), 4.85 (m, 1H), 5.04 (m, 1H), 5.43 (d, *J* = 8.3 Hz, 1H), 7.43 (dd, *J* = 8.8, 2.3 Hz, 1H), 7.55 (m, 2H), 7.76 (d, *J* = 2.0 Hz, 1H), 7.87 (m, 1H), 7.90-7.97 (m, 2H); ¹³C NMR (CDCl₃, 500 MHz) δ 13.1, 22.7, 24.1, 26.6, 33.5, 37.2, 63.9, 76.7, 85.7, 87.8, 111.4, 118.7, 121.1, 124.0, 126.0, 126.7, 127.7, 127.8, 129.5, 131.7, 133.7, 149.3, 151.8, 160.1, 172.1; MS (ESI) *m*/*z*: 494 [MH]⁺.

### Example 17: Preparation of (3aR,4S,6R,6aS)-2,2-dimethyl-6-(7-(naphthalen-1-yloxy)-5-(propylthio)-3H-[1,2,3]triazolo[4,5-d]pyrimidin-3-yl)tetrahydro-3aH-cyclopenta[d][1,3]dioxol-4-ol (ANOTAM)

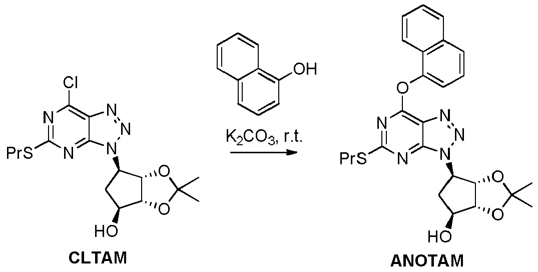

A mixture of **CLTAM** (3.09 g, 8 mmol), 1-naphthol (1.33 g, 9.2 mmol) and K₂CO₃ (1.44 g, 10.4 mmol) in 2-butanone (40 mL) was stirred 24 h at 25 °C. The reaction mixture was washed with water (2×40 mL) and evaporated under reduced pressure. The residue was recrystallized from a diisopropyl ether / ethyl acetate mixture to give **ANOTAM** as an off-white powder (2.43 g, 62%): mp 137-139 °C; ¹H NMR (CDCl₃, 500 MHz) δ 0.58 (t, *J* = 7.3 Hz, 3H), 1.24 (m, 2H), 1.34 (s, 3H), 1.54 (s, 3H), 2.34 (d, *J* = 15.4 Hz, 1 H), 2.59 (t, *J* = 7.4 Hz, 2H), 2.94 (m, 1H), 4.46 (m, 2H), 4.85 (m, 1H), 5.01 (m, 1H), 5.43 (d, *J* = 8.4 Hz, 1H), 7.41 (dd, *J* = 7.5, 0.7 Hz, 1H), 7.47 (td, *J* = 6.9, 1.2 Hz, 1H), 7.51-7.57 (m, 2H), 7.86 (m, 2H), 7.93 (d, *J* = 8.2 Hz, 1H); ¹³C NMR (CDCl₃, 500 MHz) δ 13.1, 22.6, 24.1, 26.6, 33.5, 37.2, 64.0, 76.8, 85.8, 87.8, 111.4, 118.1, 121.4, 123.8, 125.3, 126.61, 126.64, 126.7, 126.8, 128.0, 134.8, 147.8, 151.9, 160.4, 172.2; MS (ESI) *m*/*z*: 494 [MH]⁺.

### Example 18: Preparation of (3aR,4S,6R,6aS)-2,2-dimethyl-6-(7-(phenylthio)-5-(propylthio)-3H-[1,2,3]triazolo[4,5-d]pyrimidin-3-yl)tetrahydro-3aH-cyclopenta[d][1,3]dioxol-4-ol (TOTAM)

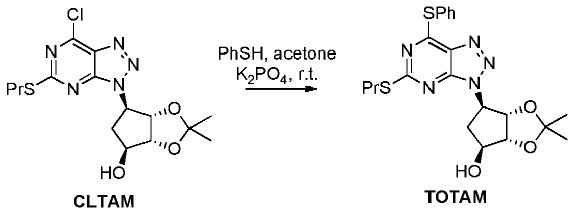

A mixture of **CLTAM** (1.0 g, 2.59 mmol), thiophenol (0.29 mL, 2.85 mmol), and K₂CO₃ (0.39 g, 2.85 mmol) in acetone (3 mL) was stirred at room temperature for 16 hours. Then volatile components were evaporated, *i*Pr₂O (10 mL) was added, salts were filtered off, and filtrate was concentrated to afford crude product, which was purified by chromatography (SiO₂, hexane:EtOAc) to give title compound as colorless oil (0.95 g, 80% yield). MS (ESI) *m*/*z*: 460 [MH]⁺.

### Example 19: Preparation of 2-(3-((3aS,4R,6S,6aR)-6-hydroxy-2,2-dimethyltetrahydro-3aH-cyclopenta[d][1,3]dioxol-4-yl)-5-(propylthio)-3H-[1,2,3]triazolo[4,5-d]pyrimidin-7-yl)isoindoline-1,3-dione (FATAM)

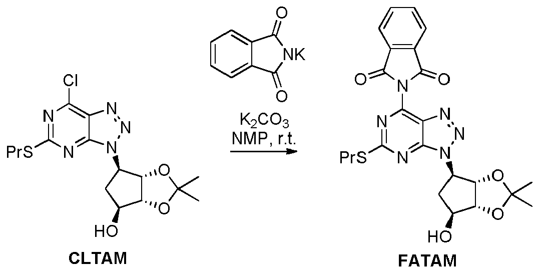

A mixture of **CLTAM** (1.54 g, 4 mmol) and potassium phthalimidate (0.85 g, 4.6 mmol) in *N-*methylpyrrolidin-2-one (20 mL) was stirred 24 h at 25 °C. The reaction mixture was diluted with water (80 mL), extracted with ethyl acetate (50 mL), the extract washed with water (2×30 mL) and evaporated under reduced pressure to give a crude product, which was further purified by flash chromatography to give **FATAM** as a crystalline solid (1.40 g, 70%): MS (ESI) *m*/*z*: 497 [MH]⁺.

### Example 20: Preparation of (3aR,4S,6R,6aS)-6-(7-(1H-imidazol-1-yl)-5-(propylthio)-3H-[1,2,3]triazolo[4,5-d]pyrimidin-3-yl)-2,2-dimethyltetrahydro-3aH-cyclopenta[d][1,3]dioxol-4-ol (IMTAM)

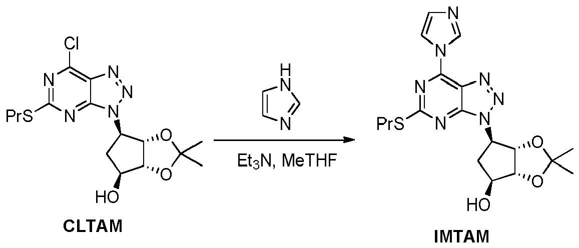

A mixture of **CLTAM** (1.16 g, 3.0 mmol), triethylamine (0.46 g, 4.5 mmol), imidazole (0.22 g, 3.3 mmol) and 2-methyltetrahydrofuran (5 mL) was stirred at 25 °C for 4 hours. Brine (10 mL) and water (10 mL) were added and the phases were separated. Organic phase was concentrated under reduced pressure and the product was purified by chromatography (SiO₂, hexane:EtOAc) to afford title compound (0.90 g, 72 % yield). ¹H NMR (CDCl₃): δ 1.10 (t, 3H; *J* = 7.4 Hz), 1.33 (s, 3H), 1.53 (s, 3H), 1.84 (m, 2H), 2.44 (m, 1H), 2.87 (m, 1 H), 3.22 (m, 2H), 4.30 (s, 1 H), 4.44 (s, 1H), 4.80 (d, 1H; *J* = 5.7 Hz), 5.18 (d, 1H; *J* = 5.7 Hz), 5.34 (m,1H), 7.23 (m, 1H), 8.31 (m, 1H), 9.09 (m, 1H).

### Example 21: Preparation of methyl 2-(((3aR,4S,6R,6aS)-6-(7-methoxy-5-(propylthio)-3H-[1,2,3]triazolo[4,5-d]pyrimidin-3-yl)-2,2-dimethyltetrahydro-3aH-cyclopenta[d][1,3]dioxol-4-yl)oxy)acetate (MOTAME)

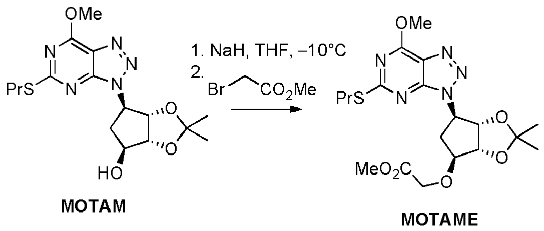

To a solution of **MOTAM** (1.0 g, 2.62 mmol) in dry THF (10 mL) NaH (60%, 0.12 g, 2.88 mmol) was added at -10°C and stirred for 15 min, then methyl bromoacetate (0.27 mL, 2.88 mmol) was added at -10°C. Resulting reaction mixture was stirred at -10°C for 2 h. Water was added (50 mL), and product was extracted to MeTHF (3 x 20 mL). Combined organic layers were dried over MgSO₄, and concentrated to afford crude product, which was then purified by chromatography (SiO₂, hexane:EtOAc) to afford colorless oil (0.95 g, 80% yield). ¹H NMR (CDCl₃, 500 MHz) δ 1.09 (t, *J* = 7.3 Hz, 3H), 1.35 (s, 3H), 1.55 (s, 3H), 1.83 (m, 2H), 2.74 (m, 2H), 3.19 (m, 2H), 3.73 (s, 3H), 4.13 (s, 3H), 4.22 (s, 3H), 4.83 (dd, *J* = 6.9, 2.6 Hz, 1H), 5.16 (m, 1H), 5.31 (s, 3H), 5.50 (dd, *J* = 6.9, 3.8 Hz, 1H); MS (ESI) *m*/*z*: 454 [MH]⁺.

### Example 22: Preparation of isopropyl 2-(((3aR,4S,6R,6aS)-6-(7-methoxy-5-(propylthio)-3H-[1,2,3]triazolo[4,5-d]pyrimidin-3-yl)-2,2-dimethyltetrahydro-3aH-cyclopenta[d][1,3]dioxol-4-yl)oxy)acetate (MOTAMEI)

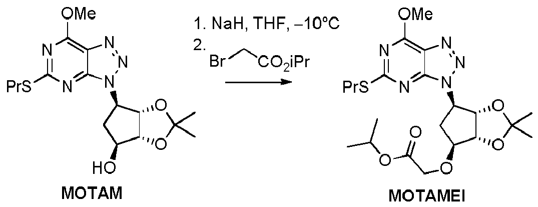

To a solution of **MOTAM** (3.81 g, 10.0 mmol) in dry THF (10 mL) NaH (60%, 0.44 g, 11.0 mmol) was added at -10°C and stirred for 15 min, then isopropyl bromoacetate (1.42 mL, 11.0 mmol) was added at -10°C. Resulting reaction mixture was stirred at -10°C for 16 h. Acetic acid (5 mL) and water (50 mL) were added, and product was extracted to MeTHF (3 x 20 mL). Combined organic layers were dried over MgSO₄, and concentrated to afford crude product, which was then purified by chromatography (SiO₂, hexane:EtOAc) to afford colorless oil (3.88 g, 81% yield). MS (ESI) *m*/*z*: 482 [MH]⁺.

### Example 23: Preparation of methyl 2-(((3aR,4S,6R,6aS)-6-(7-(benzyloxy)-5-(propylthio)-3H-[1,2,3]triazolo[4,5-d]pyrimidin-3-yl)-2,2-dimethyltetrahydro-3aH-cyclopenta[d][1,3]dioxol-4-yl)oxy)acetate (BOTAME)

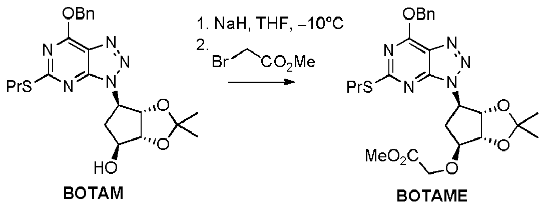

To a solution of **BOTAM** (1.0 g, 2.19 mmol) in dry THF (10 mL) NaH (60%, 105 mg, 2.63 mmol) was added at -10°C and stirred for 15 min, then methyl bromoacetate (0.25 mL, 2.63 mmol) was added at -10°C. Resulting reaction mixture was stirred at -10°C for 2 h. Acetic acid (1 mL) and water (50 mL) were added, and product was extracted to MeTHF (3 x 20 mL). Combined organic layers were dried over MgSO₄, and concentrated to afford crude product, which was then purified by chromatography (SiO₂, hexane:EtOAc) to afford colorless syrup (0.87 g, 78% yield). MS (ESI) *m*/*z*: 530 [MH]⁺.

### Example 24: Preparation of 2-(((3aR,4S,6R,6aS)-6-(7-methoxy-5-(propylthio)-3H-[1,2,3]triazolo [4,5-d]pyrimidin-3-yl)-2,2-dimethyltetrahydro-3aH-cyclopenta[d][1,3]dioxol-4-yl)oxy)ethanol (MOTAMA)

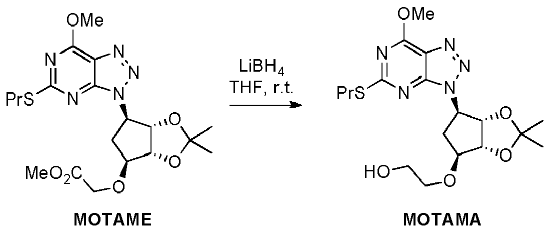

To a solution of **MOTAME** (0.85 g, 1.87 mmol) in dry THF (15 mL) at 0°C LiBH₄ (90 mg, 4.12 mmol) was added, and resulting reaction mixture was stirred at room temperature for 16 h. Reaction was then quenched by slow addition of AcOH (5 mL), water was added (50 mL), and product was extracted to MeTHF (3 x 30 mL). Combined organic layers ware dried over MgSO₄, and then concentrated to afford crude product, which was purified by chromatography (SiO₂, hexane:EtOAc) to afford title compound as colorless oil (0.73 g, 92% yield). ¹H NMR (CDCl₃, 500 MHz) δ 1.09 (t, *J* = 7.3 Hz, 3H), 1.37 (s, 3H), 1.55 (s, 3H), 1.83 (m, 2H), 2.23 (m, 1H), 2.52 (m, 1H), 2.68 (m, 1H), 3.20 (m, 2H), 3.50 (m, 1H), 3.52-3.65 (m, 3H), 4.03 (m, 1H), 4.22 (s, 3H), 4.89 (m, 1H), 5.20 (m, 1H), 5.56 (m, 1H); MS (ESI) *m*/*z*: 426 [MH]⁺.

### Example 25: Preparation of 2-(((3aR,4S,6R,6aS)-6-(7-methoxy-5-(propylthio)-3H-[1,2,3]triazolo [4,5-d]pyrimidin-3-yl)-2,2-dimethyltetrahydro-3aH-cyclopenta[d][1,3]dioxol-4-yl)oxy)ethanol (MOTAMA) through one-pot reaction starting from MOTAM

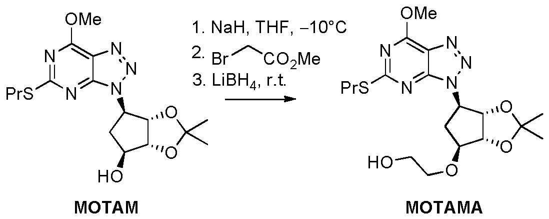

To a solution of **MOTAM** (1.8 g, 4.72 mmol) in dry THF (20 mL) NaH (60%, 0.23 g, 5.66 mmol) is added at -10°C and stirred for 15 min, then methyl bromoacetate (0.54 mL, 5.66 mmol) is added at -10°C. Resulting reaction mixture is stirred at -10°C for 2 h, then LiBH₄ (0.21 g, 9.44 mmol) was added, and resulting reaction mixture was stirred at room temperature for 2 h. Reaction was then quenched by slow addition of AcOH (2 mL), water was added (100 mL), and product was extracted to MeTHF (3 x 30 mL). Combined organic layers were dried over MgSO₄, and then concentrated to afford crude product, which was purified by chromatography (SiO₂, hexane:EtOAc) to afford title compound as colorless oil (1.52 g, 76% yield). ¹H NMR (CDCl₃, 500 MHz) δ 1.09 (t, *J* = 7.3 Hz, 3H), 1.37 (s, 3H), 1.55 (s, 3H), 1.83 (m, 2H), 2.23 (m, 1H), 2.52 (m, 1H), 2.68 (m, 1H), 3.20 (m, 2H), 3.50 (m, 1H), 3.52-3.65 (m, 3H), 4.03 (m, 1H), 4.22 (s, 3H), 4.89 (m, 1H), 5.20 (m, 1H), 5.56 (m, 1H); MS (ESI) *m*/*z*: 426 [MH]⁺.

### Example 26: Preparation of 2-(((3aR,4S,6R,6aS)-6-(7-(benzyloxy)-5-(propylthio)-3H-[1,2,3]triazolo[4,5-d]pyrimidin-3-yl)-2,2-dimethyltetrahydro-3aH-cyclopenta[d][1,3]dioxol-4-yl)oxy)ethanol (BOTAMA)

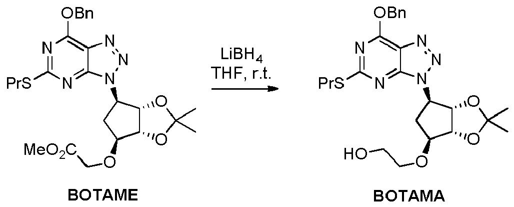

To a solution of **BOTAME** (0.80 g, 1.51 mmol) in dry THF (10 mL) at room temperature LiBH₄ (66 mg, 3.02 mmol) was added, and resulting reaction mixture was stirred at room temperature for 1 h. Reaction was then quenched by slow addition of AcOH (2 mL), water was added (20 mL), and product was extracted to EtOAc (3x10 mL). Combined organic layers were dried over MgSO₄, and then concentrated to afford crude product, which was purified by chromatography (SiO₂, hexane:EtOAc) to afford title compound as colorless oil (0.61 g, 81% yield). ¹H NMR (CDCl₃, 500 MHz) δ 1.09 (t, *J* = 7.3 Hz, 3H), 1.37 (s, 3H), 1.55 (s, 3H), 1.83 (m, 2H), 2.23 (m, 1H), 2.52 (m, 1H), 2.68 (m, 1H), 3.20 (m, 2H), 3.50 (m, 1H), 3.52-3.65 (m, 3H), 4.03 (m, 1H), 4.22 (s, 3H), 4.89 (m, 1H), 5.20 (m, 1H), 5.56 (m, 1H); MS (ESI) *m*/*z*: 502 [MH]⁺.

### Example 27: Preparation of methyl 2-(((3aR,4S,6R,6aS)-6-(7-(((1R,2S)-2-(3,4-difluorophenyl)cyclopropyl)amino)-5-(propylthio)-3H-[1,2,3]triazolo[4,5-d]pyrimidin-3-yl)-2,2-dimethyltetrahydro-3aH-cyclopenta[d][1,3]dioxol-4-yl)oxy)acetate (CPATAME)

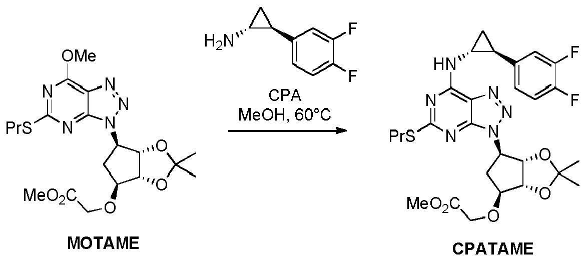

A solution of **MOTAME** (1.0 g, 2.21 mmol) and **CPA** (0.41 g, 2.43 mmol) in MeOH (10 mL) was stirred at 60°C until TLC showed total conversion (several days). Water was added (50 mL), and product was extracted to MeTHF (3 x 10 mL). Combined organic layers were dried over MgSO₄, and concentrated to afford crude product, which was then purified by chromatography (SiO₂, hexane:EtOAc) to afford colorless syrup (0.26 g, 20% yield). MS (ESI) *m*/*z*: 591 [MH]⁺. **Example 28:** Preparation of 2-(((3a*R*,4*S*,6*R*,6a*S*)-6-(7-(((1R,2S)-2-(3,4-difluorophenyl) cyclopropyl)amino)-5-(propylthio)-3*H*-[1,2,3]triazolo[4,5-d]pyrimidin-3-yl)-2,2-dimethyltetrahydro-3a*H*-cyclopenta[*d*][1,3]dioxol-4-yl)oxy)ethanol **(CPATAMA)**

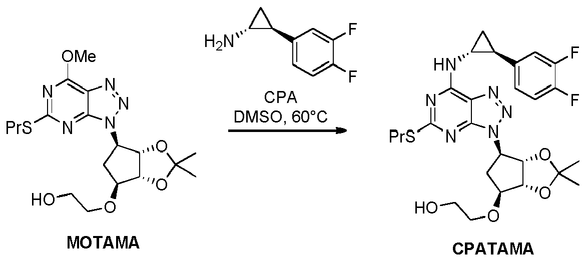

To a solution of **MOTAMA** (0.70 g, 1.65 mmol) in dry DMSO (5 mL) at room temperature **CPA** (0.29 g, 1.73 mmol) was added and reaction mixture was stirred at 60°C for 16 h. Water was added (50 mL), and product was extracted to MeTHF (3 x 10 mL). Combined organic layers were dried over MgSO₄, and concentrated to afford crude product, which was then purified by chromatography (SiO₂, hexane:EtOAc) to afford colorless syrup (0.72 g, 78% yield). MS (ESI) *m*/*z*: 563 [MH]⁺.

**CPATAMA** was also prepared from **BOTAMA** according to the same procedure as described above.

### Example 29: Preparation of (1S,2S,3R,5S)-3-(7-(((1R,2S)-2-(3,4-difluorophenyl)cyclopropyl) amino)-5-(propylthio)-3H-[1,2,3]triazolo[4,5-c]pyrimidin-3-yl)-5-(2-hydroxyethoxy)cyclopentane-1,2-diol (TCG)

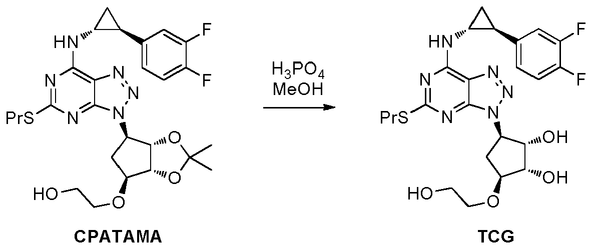

To a solution of **CPATAMA** (0.21 g, 0.38 mmol) in MeOH (10 mL) at room temperature *ortho*phosphoric acid (85%, 1.5 mL) was slowly added. Resulting reaction mixture was stirred at room temperature for 24 h, then water was added (20 mL), and reaction mixture was neutralized with 1 M NaOH. Product was extracted to EtOAc (5 x 10 mL), combined organic phases were dried over Na₂SO₄, then concentrated to afford crude product, which was purified by chromatography (SiO₂, EtOAc) to afford title compound as a white powder (0.18 g, 90 % yield). ¹⁹F NMR (CD₃OD, 470.5 MHz) δ -141.9--142.1 (m, 1F), -145.6--145.9 (m, 1F); MS (ESI) *m*/*z*: 523 [MH]⁺.

## Claims

1. A process for the preparation of a compound of formula **VIII** 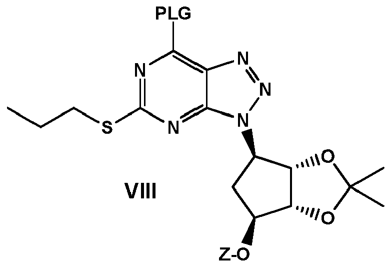 wherein PLG is a protecting-leaving group, and Z is hydroxyethyl or a group convertible to hydroxyethyl,
the process comprising the steps of:
(i) providing a compound of formula VII 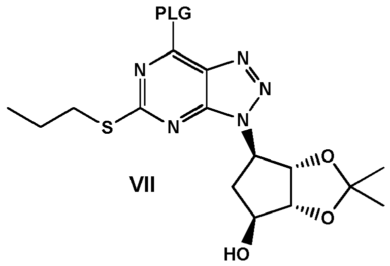 wherein PLG is defined as above, and
(ii) O-alkylating the compound of formula VII to obtain the compound of formula VIII,
wherein said protecting-leaving group PLG is both, capable of acting as a protecting group in the O-alkylation reaction (ii) and capable of acting as a leaving group when it will be subjected to a nucleophilic substitution reaction.

2. The process according to claim 1, wherein the compound of formula **VII** is prepared by
(i) providing a compound of formula **V** 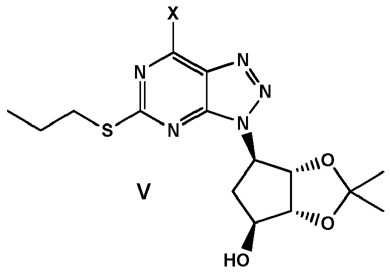 wherein X is Cl or Br, and
(ii) substituting X with PLG by reaction of a reagent PLG-H in the presence of a base, to obtain a compound of formula **VII**.

3. The process according to claim 2, wherein the compound of formula V is prepared by
(i) providing a compound of formula I' 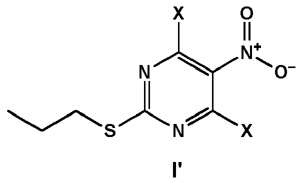 wherein X is Cl or Br,
(ii) reacting the compound of formula I' with a compound of formula III 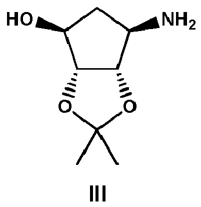 to obtain a compound of formula **IV'** 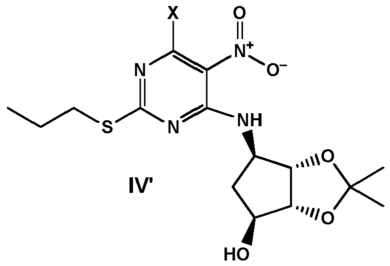 wherein X is as defined above,
(iii) reducing the nitro group of the compound of formula **IV'** to obtain a compound of formula **IV"** 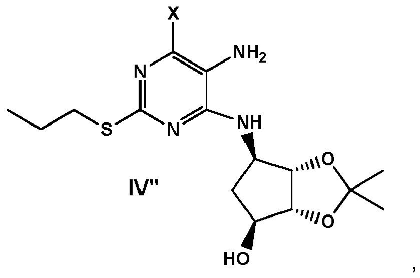 and
(iv) converting the compound of formula **IV"** into the compound of formula **V** by nitrosation.

4. The process according to claim 1, wherein the compound of formula **VII** is prepared by
(i) providing a compound of formula **VI"** 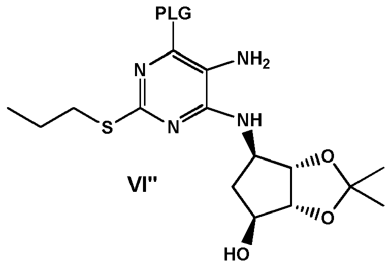 wherein PLG is defined as above, and
(ii) converting the compound of formula **VI'** into the compound of formula **VII** by nitrosation.

5. The process according to claim 4, wherein the compound of formula **VI"** is prepared by comprising the steps of
either
(0-1) providing a compound of formula **IV"** 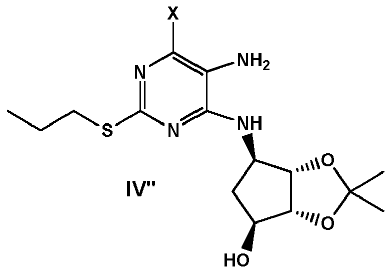 wherein X is Cl or Br,
(0-2) substituting X with PLG by reaction of a reagent PLG-H in the presence of a base, to obtain a compound of formula **VI"**
or
(0-1') providing a compound of formula **VI'** 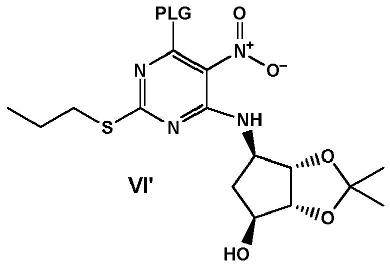
(0-2') reducing the nitro group of the compound of formula **VI'** to obtain a compound of formula **VI"**
or
(0-1 ") providing a compound of formula **II"** 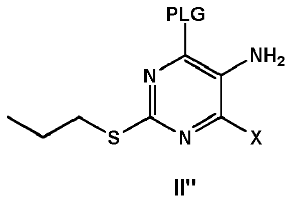 wherein X is Cl or Br,
(0-2") reacting the compound of formula **II"** with a compound of formula **III** 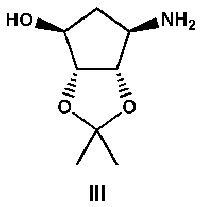 to obtain a compound of formula **VI".**

6. The process according to claim 5, wherein the compound of formula **IV"** is prepared by comprising the steps of
(i) providing a compound of formula **I'** 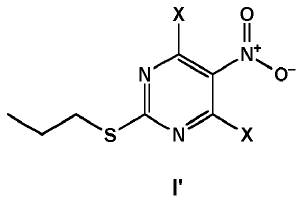 wherein X is Cl or Br,
(ii) reacting a compound of formula **I'** with a compound of formula **III** 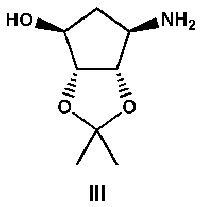 to obtain a compound of formula IV' 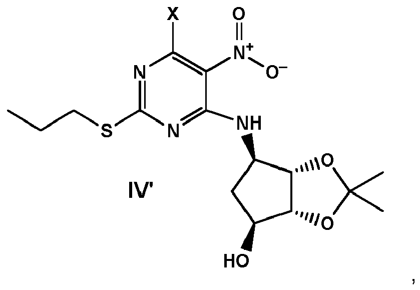 and
(iii) reducing the nitro group of the compound of formula **IV'** to obtain a compound of formula **IV"**.

7. The process according to claim 5, wherein the compound of formula **VI'** is prepared by comprising the steps of
either
(0-1) providing a compound of formula **I'** 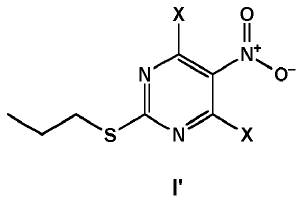
wherein X is Cl or Br,
(0-2) reacting the compound of formula **I'** with a compound of formula **III** 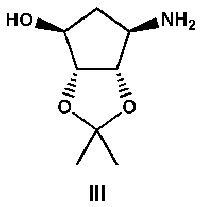 to obtain a compound of formula IV' 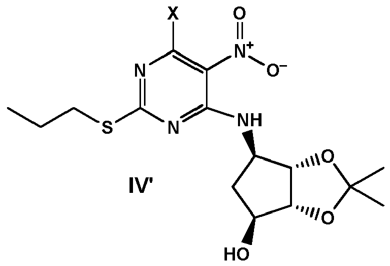 wherein X is as defined above, and
(0-3) substituting X with PLG by reaction of a reagent PLG-H in the presence of a base, to obtain a compound of formula VI'
or
(0-1') providing a compound of formula I' 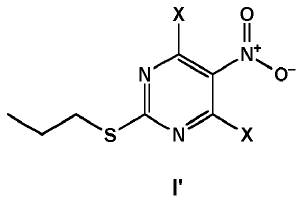 wherein X is Cl or Br,
(0-2') substituting X with PLG by reaction of a reagent PLG-H in the presence of a base, to obtain a compound of formula II' 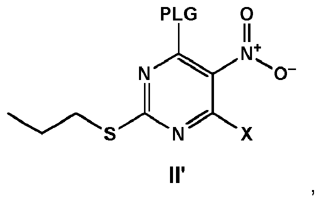 and
(0-3') reacting the compound of formula **II'** with a compound of formula **III** 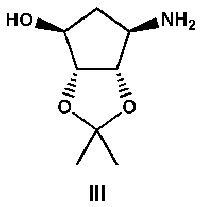 **to** obtain a compound of formula **VI'.**

8. The process according to claim 5, wherein the compound of formula **II"** is prepared by comprising the steps of
either
(0-1) providing a compound of formula **I'** 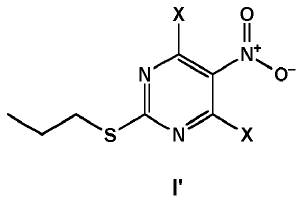 wherein X is Cl or Br,
(0-2) substituting X with PLG by reaction of a reagent PLG-H in the presence of a base, to obtain a compound of formula **II'** 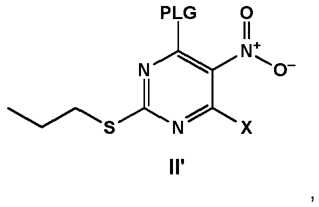 and
(0-3) reducing the nitro group of the compound of formula **II'** to obtain a compound of formula **II"**
or
(0-1') providing a compound of formula **I'** 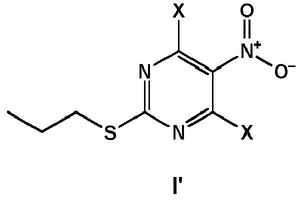 wherein X is Cl or Br,
(0-2') reducing the nitro group of the compound of formula **I'** to obtain a compound of formula **I"** 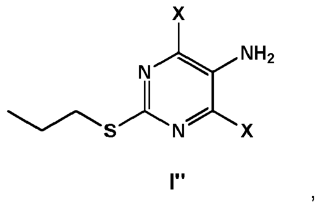 and
(0-3') substituting X with PLG by reaction of a reagent PLG-H in the presence of a base, to obtain a compound of formula **II"**.

9. The process according to claims 4, 5 and 7, wherein steps (0-1') to (0-3') of claim 7, steps (0-1') to (0-2') of claim 5 and steps (i) to (ii) of claim 4, are carried out in one pot.

10. The process according to any one of claims 1 to 9, wherein PLG is selected from the group consisting of linear or branched C₁-C₆-alkoxy groups, optionally substituted with one or more aryl, heteroaryl, halo, C₁-C₄-alkoxy, C₁-C₄-alkylthio; aryloxy or substituted aryloxy; C₈-C₂₀-alkylthio, unsubstituted or substituted arylthio or heteroarylthio; N-azolyl groups, selected from unsubstituted or substituted 1-imidazolyl, 1-pyrrolyl, 1-pyrazolyl, 1-indolyl, 1-(1,2,3-triazolyl), 1-(1,2,4-triazolyl), 4-(1,2,4-triazolyl), 1-tetrazolyl, 2-tetrazolyl, 1-benzopyrazolyl, 1-benzimidazolyl, 1-benzotriazolyl, 5-carbazolyl, 4-aza, 5-aza, 6-aza, 7-aza, 4,5-diaza, 4,6-diaza, 4,7-diaza, 5,6-diaza, 5,7-diaza, or 6,7-diaza derivatives of 1-benzopyrazolyl, 1-benzimidazolyl, or 1-benzotriazolyl; N-amidyl groups selected from unsubstituted or substituted N-aryl-N-(C₁-C₆-alkanoyl)amino, 3-(2-oxo-1,3-oxazolidinyl), 3-(2-oxo-1,3-benzoxazolidinyl), 2-oxo-1-(1,2-dihydropyridyl), 2-oxo-1-(1,2-dihydroquinolyl), 2-oxo-1-(1,2-dihydroquinazolyl); 1-benzotriazolyloxy; azido, and cyano.

11. The process according to claim 10, wherein C₁-C₆-alkoxy group is methoxy, aryloxy group is phenoxy and N-azolyl group is 1-imidazolyl.

12. A process for the preparation of a compound of formula **XI** 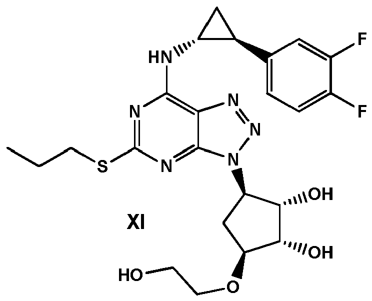 **or** a salt thereof,
comprising the steps of
(i) preparing a compound of formula **VIII** according to any one of claims 1 to 11 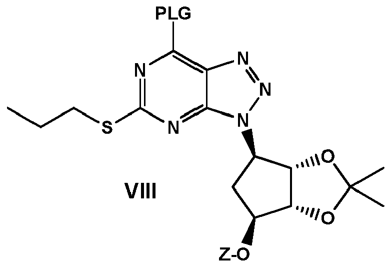
wherein PLG is a protecting-leaving group, and Z is hydroxyethyl or a group convertible to hydroxyethyl,
(ii) reacting a compound of formula **VII** with a compound of formula **IX** 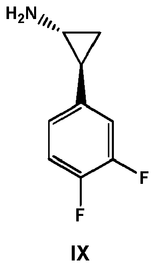
(iii) carrying out deprotection reaction to remove the vicinal hydroxyl protecting group at the pentane ring,
(iv) optionally converting group Z, if not hydroxyethyl, into hydroxyethyl, and
(v) optionally forming a salt of the compound of formula **XI.**

13. A process for the preparation of a pharmaceutical composition comprising a compound of formula **XI** or a salt thereof 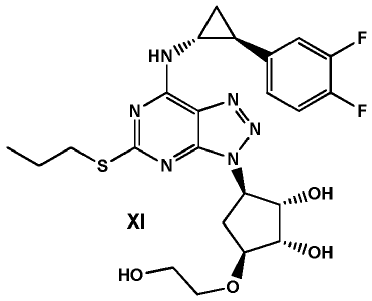 comprising the steps of:
(i) preparing a compound of formula **XI** or a salt thereof according to claim 12, and
(ii) mixing the compound of formula **XI** or a salt thereof with a pharmaceutically acceptable carrier and/or excipient.

14. A compound of formula **II** 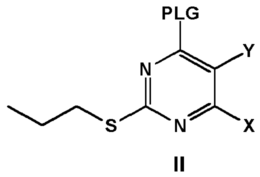 wherein PLG is a protecting-leaving group, excluding Cl and Br, X is Cl or Br, and Y is NO₂ or NH₂.

15. A compound of formula **VI** 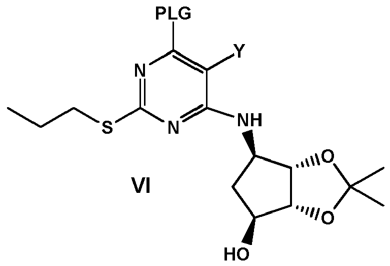 wherein PLG is a protecting-leaving group, excluding Cl and Br, and Y is NO₂ or NH₂.

16. A compound of formula **VIII** 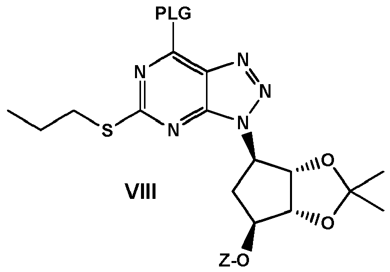 wherein PLG is a protecting-leaving group, excluding Cl and Br,
and Z is hydrogen, hydroxyethyl or a group convertible to hydroxyethyl.

17. The compound according to claim 16, wherein Z is selected from hydrogen, hydroxyethyl and (CH₃)O₂C-(CH₂)-.

18. Use of a compound as defined in any one of claims 14 to 17 in the preparation of ticagrelor.
